(19) Europäisches Patentamt
European Patent Office
Office européen des brevets

(11) EP 3 564 367 A1

(12) EUROPEAN PATENT APPLICATION
published in accordance with Art. 153(4) EPC

(43) Date of publication:
**06.11.2019 Bulletin 2019/45**

(21) Application number: **17886849.3**

(22) Date of filing: **27.12.2017**

(51) Int Cl.:
***C12N 15/09*** (2006.01) ***C12N 1/15*** (2006.01)
***C12N 1/19*** (2006.01) ***C12N 1/21*** (2006.01)
***C12N 5/10*** (2006.01) ***C12P 21/00*** (2006.01)
***C12P 21/08*** (2006.01) ***C12Q 1/04*** (2006.01)
***C12Q 1/68*** (2018.01)

(86) International application number:
**PCT/JP2017/046939**

(87) International publication number:
**WO 2018/124189 (05.07.2018 Gazette 2018/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **28.12.2016 JP 2016256279**

(71) Applicant: **Kyowa Hakko Kirin Co., Ltd.**
**Tokyo 100-8185 (JP)**

(72) Inventors:
- **YAMAMOTO, Koichi**
  **Tokyo 100-8185 (JP)**
- **MATSUMOTO, Yuichi**
  **Tokyo 100-8185 (JP)**
- **YAMAGUCHI, Keina**
  **Tokyo 100-8185 (JP)**
- **FUJII, Hiroto**
  **Tokyo 100-8185 (JP)**
- **SUZAWA, Toshiyuki**
  **Tokyo 100-8185 (JP)**

(74) Representative: **Hoffmann Eitle**
**Patent- und Rechtsanwälte PartmbB**
**Arabellastraße 30**
**81925 München (DE)**

(54) **METHOD OF SELECTING CELLS**

(57) The purpose of the present invention is to provide a method of selecting cell lines in which the reduction of a recombinant protein has been suppressed. The present invention pertains to a method of selecting cells, said method comprising a first step in which the intracellular expression level of at least one gene selected from among genes having any one of the base sequences represented by SEQ ID NO: 1-16 and orthologs thereof is measured, and a second step in which the expression level achieved by suppressing the reduction of a recombinant protein is evaluated on the basis of the difference determined by comparing the gene expression level measured in the first step and a control value for the gene expression level.

EP 3 564 367 A1

## Description

Technical Field

The present invention relates to a method for selecting a cell, and more specifically relates to a method for selecting a cell capable of suppressing reduction of a recombinant protein.

Background Art

With the recent development of gene recombination techniques, biopharmaceutical products such as antibodies have begun to be widely supplied. These biopharmaceutical products are produced by using a production cell prepared by introducing an expression vector containing a base sequence encoding a recombinant protein (hereinafter sometimes also referred to as "target protein" for distinguishing it from a protein translated based on an endogenous gene and secreted from the same cell) into a host cell such as E. coli, yeast, an insect cell, a plant cell, or an animal cell.

In a step generally used as a step of producing biologics (protein drugs), at first, production cells are cultured under appropriate conditions, and allowed to secrete a target protein in a culture broth. The culture broth containing the target protein is subjected to purification after removing the production cells which are no longer needed.

However, in the culture broth before purification, many proteins having various enzyme activities derived from the production cells are present other than the target protein. Thus, due to the activities of those proteins, the target protein may be sometimes degraded or denatured. Further, even if the culture broth containing the target protein is purified, depending on the purification degree, proteins derived from the production cells remain, and therefore, the target protein may be sometimes degraded or denatured.

As one of the causes of degrading or denaturing the target protein, a reducing activity of thioredoxin reductase or the like endogenously present in the production cells is known. Specifically, a disulfide bond contained in the protein is reduced by thioredoxin reductase, and therefore, a high-order structure necessary for expressing the activity of the protein is lost (Non-Patent Document 1).

In order to suppress this reducing activity causing degradation or denaturation of the target protein, an attempt to add a thioredoxin reductase inhibitor or an oxidizing agent to the culture broth, or to incorporate air sparging or the like to the culture broth in the production step has been made (Patent Document 1, and Non-Patent Documents 2 and 3).

Further, for the purpose of establishing production cells in which a reducing activity is suppressed, an attempt to produce a protein using cells in which the thioredoxin reductase gene is knocked down has been made. However, the establishment of production cells focusing on such gene has not yet been put into practical use because there is a problem that the growth of the production cells is significantly deteriorated due to the knockdown, or the like (Non-Patent Document 4).

Further, for selecting production cells in which a reducing activity is suppressed, a method other than evaluation of a reducing activity imitating the production processes (Non-Patent Document 1) has not been known at present, and it is not easy to evaluate and select a large number of cells.

As described above, for suppressing the reducing activity causing degradation or denaturation of the target protein, many methods targeted at culture or a step after that during the production have been proposed. However, at present, only a few methods focus on a step of establishing production cells, such as improvement or selection of a host cell or a genetically modified cell or the like.

Citation List

Patent Literature

Patent Document 1: JP-A-2014-129358

Non Patent Literature

Non-Patent Document 1: Biotechnology and Bioengineering, 622-632, 7(4), 2010
Non-Patent Document 2: Biotechnology and Bioengineering, 452-461, 106(3), 2010
Non-Patent Document 3: Bioengineering and Biotechnology, 734-742, 112(4), 2015
Non-Patent Document 4: Journal of Biotechnology, 261-267, 157, 2012

## Summary of Invention

### Problem to Be Solved by the Invention

As described above, in order to produce a protein using a recombinant host cell, a cell in which degradation or denaturation of a target protein does not occur, and above all, particularly, a cell in which reduction of a target protein does not occur is needed for a use in the production.

Therefore, an object of the present invention is to provide a method for selecting a cell in which a reducing activity against a recombinant protein (target protein) is suppressed.

### Means for Solving the Problems

The present inventors made intensive studies for achieving the above object, and as a result, they surprisingly found that the strength of the reducing activity causing degradation or denaturation of a target protein varies in each cell. Then, they specified a gene group whose association with this reducing activity has totally not been suggested, and found a correlation between the expression level of the gene and the reducing activity of cells. Further, they found that by selecting a cell using the expression of the gene or the expression level of a protein encoded by the gene as an index, when the target protein is produced using the cell, the reducing activity for the target protein can be suppressed, and thus completed the present invention.

That is, the present invention relates to the following [1] to [15].

[1] A method for selecting a cell, comprising the following first step and second step:

a first step: measuring the expression level of a gene in a cell, the gene being at least one gene selected from genes comprising any one of the base sequences represented by SEQ ID NOS: 1 to 16 or orthologous genes thereof; and

a second step: comparing the expression level of the gene measured in the first step with a control value of the expression level of the gene in a control cell or comparing the expression level of the gene in the respective cells, and evaluating the expression level capable of suppressing reduction of a recombinant protein based on a difference therebetween.

[2] A method for selecting a cell, comprising the following first step and second step:

a first step: measuring the expression level of a protein encoded by at least one gene selected from genes comprising any one of the base sequences represented by SEQ ID NOS: 1 to 16 or orthologous genes thereof; and

a second step: comparing the expression level of the protein encoded by the gene measured in the first step with a control value of the expression level of the protein encoded by the gene in a control cell or comparing the expression level of the protein encoded by the gene in the respective cells, and evaluating the expression level capable of suppressing reduction of a recombinant protein based on a difference therebetween.

[3] A method for selecting a cell, comprising the following first step and second step:

a first step: measuring the expression level of a gene in a cell, the gene being at least one gene selected from genes comprising any one of the base sequences represented by SEQ ID NOS: 1 to 16 or orthologous genes thereof; and

a second step: calculating and comparing a relative expression level and selecting a cell according to the following procedures (a) to (d);

(a) calculating a relative quantitative value by dividing the expression level of the gene in a test cell measured in the first step by the expression level of a standard gene in the test cell

$$\text{a relative quantitative value} = (\text{the expression level of the gene in a test cell measured in the first step}) / (\text{the expression level of a standard gene}),$$

(b) calculating a control value by dividing the expression level of the gene in a control cell by the expression level of the standard gene in the control cell

a control value = (the expression level of the gene in a control cell) / (the expression level of the standard gene),

(c) calculating a relative expression level by dividing the relative quantitative value for the test cell calculated in (a) by the control value calculated in (b)

a relative expression level = [the relative quantitative value for the test cell calculated in (a)] / [the control value calculated in (b)],

and

(d) comparing the relative quantitative values calculated in (a) or the relative expression level calculated in (c) among the respective test cells, and thereby selecting a cell in which each value is high or low.

[4] The method according to [3], wherein in the second step (d), the cell in which the relative quantitative value or the relative expression level is high is a cell in which the relative quantitative value or the relative expression level is twice or more the control value, or the cell in which the relative quantitative value or the relative expression level is low is a cell in which the relative quantitative value or the relative expression level is 1/2 or less of the control value.

[5] The method according to any one of [1] to [4], comprising introducing at least one gene selected from genes comprising any one of the base sequences represented by SEQ ID NOS: 1 to 9 or orthologous genes thereof into the cell, thereby transforming the cell before the first step.

[6] The method according to any one of [1] to [5], comprising knocking down or knocking out at least one gene selected from genes comprising any one of the base sequences represented by SEQ ID NOS: 10 to 16 or orthologous genes thereof in a cell before the first step.

[7] The method according to any one of [1] to [6], comprising introducing a gene encoding a recombinant protein into the cell, thereby transforming the cell before the first step.

[8] A method for obtaining a cell producing a recombinant protein, comprising selecting a cell by the method according to any one of [1] to [7].

[9] A method for producing a recombinant protein using a cell selected or obtained by the method according to any one of [1] to [8] or a cell.

[10] The method according to any one of [1] to [9], wherein the cell is a cell derived from a mammalian cell.

[11] The method according to any one of [1] to [10], wherein the gene is Pletl gene.

[12] The method according to any one of [1] to [11], wherein the recombinant protein is an antibody.

[13] A cell capable of suppressing reduction of a recombinant protein, wherein at least one gene selected from genes comprising any one of the base sequences represented by SEQ ID NOS: 1 to 9 or orthologous genes thereof is introduced to transform the cell.

[14] A cell capable of suppressing reduction of a recombinant protein, wherein at least one gene selected from genes comprising any one of the base sequences represented by SEQ ID NOS: 10 to 16 or orthologous genes thereof is knocked down or knocked out.

[15] A method for increasing probability of obtaining a cell capable of suppressing reduction of a recombinant protein by using at least one gene selected from genes comprising any one of the base sequences represented by SEQ ID NOS: 1 to 16 or orthologous genes thereof as an index.

## Effects of the Invention

According to the method of the present invention, from a cell population having been subjected to gene recombination, a cell in which a reducing activity against a target protein is suppressed can be easily selected with high probability. By combining the method with evaluation of an index other than susceptibility to reduction, such as productivity of a protein or the like, a gene recombinant protein-producing cell having a desired property for producing a pharmaceutical product can be efficiently selected.

Further, according to the method of the present invention, a cell in which a reducing activity against a target protein is suppressed can be obtained. By using this cell, a target protein-producing cell in which a reducing activity against the target protein is suppressed can be obtained.

## Brief Description of Drawings

Fig. 1 is a figure showing the results of capillary electrophoresis showing that an antibody produced by A#1 strain is not reduced and is stably present as the whole antibody, and that an antibody produced by A#2 strain is degraded over time by reduction.

Fig. 2 is a figure showing changes in the relative expression level of Plet1 gene in 80 days of subculture period of antibody C-producing cells.

Embodiments for Carrying Out the Invention

A first embodiment of the present invention is a method for selecting a cell in which a reducing activity for a recombinant protein (target protein) is suppressed, comprising the following first step and second step:

a first step: measuring the expression level of a gene in a cell, the gene being at least one gene selected from genes comprising any one of the base sequences represented by SEQ ID NOS: 1 to 16 or orthologous genes thereof; and
a second step: comparing the expression level of the gene measured in the first step with a control value of the expression level of the gene, and evaluating the expression level capable of suppressing reduction of a recombinant protein based on a difference therebetween.

The present inventors compared, in cells producing an antibody, a cell in which the antibody is not easily reduced and a cell in which the antibody is easily reduced through a gene expression difference analysis using a microarray, and found that the expression level of genes comprising a base sequence represented by any of SEQ ID NOS: 1 to 16 in the cell is greatly different between the cell in which the antibody is not easily reduced and the cell in which the antibody is easily reduced.

The base sequences represented by SEQ ID NOS: 1 to 16 are base sequences registered in Affimetrix or NCBI (National Center for Biotechnology Information), and a gene comprising any one of these or an orthologous gene thereof can be obtained by searching database based on the gene name or a partial sequence from the web page of Affimetrix (https://www.affymetrix.com/analysis/netaffx/xmlquery_ex.affx?netaffx=wtgene_transcript ) or the web page of NCBI (URL;http://www.ncbi.nlm.nih.gov). On the web page of Affimetrix, it is registered as ID: 18083239. Incidentally, the "orthologous gene" refers to an analogous gene encoding a protein having a homologous function present in a different organism.

The base sequences represented by SEQ ID NOS: 1, 4, 7, 8, and 10 to 16 are base sequences of Pletl gene, matrilin 4 gene, G-protein-coupled receptor 133 gene, tenascin C gene, collagen alpha-1(III) chain gene, glutathione S-transferase alpha-3 gene, calcium/calmodulin-dependent 3',5'-cyclic nucleotide phosphodiesterase 1C gene, anthrax toxin receptor 1 gene, gastrokine-1 gene, tumor necrosis factor ligand superfamily member 9 gene, and collagen, type VI, $\alpha$2 gene, respectively.

The "Pletl gene" means a gene encoding Pletl. Pletl is placenta-expressed transcript 1 protein expressed in placenta and is a protein comprising an amino acid sequence represented by SEQ ID NO: 17. Pletl is present in various types of mammals, and a gene encoding Pletl derived from any of various mammals can be used, however, it is preferred to use a gene of a mammal from which a cell to be used as a host cell is derived. As the Pletl gene, for example, Pletl gene of a Chinese hamster comprising a base sequence represented by SEQ ID NO: 1 is exemplified.

The "matrilin 4 gene" means a gene encoding matrilin 4 (Matn4). Matrilin 4 is a protein comprising an amino acid sequence represented by SEQ ID NO: 18. Matrilin 4 is present in various types of mammals, and a gene encoding matrilin 4 derived from any of various mammals can be used, however, it is preferred to use a gene of a mammal from which a cell to be used as a host cell is derived. As the matrilin 4 gene, for example, matrilin 4 gene of a Chinese hamster comprising a base sequence represented by SEQ ID NO: 4 is exemplified.

The "G-protein-coupled receptor 133 gene" means a gene encoding G-protein-coupled receptor 133. The G-protein-coupled receptor 133 gene is a protein comprising an amino acid sequence represented by SEQ ID NO: 19. The G-protein-coupled receptor 133 gene is present in various types of mammals, and a gene encoding G-protein-coupled receptor 133 gene derived from any of various mammals can be used, however, it is preferred to use a gene of a mammal from which a cell to be used as a host cell is derived. As the G-protein-coupled receptor 133 gene, for example, G-protein-coupled receptor 133 gene of a Chinese hamster comprising a base sequence represented by SEQ ID NO: 7 is exemplified.

The "tenascin C gene" means a gene encoding tenascin C. Tenascin C is a protein comprising an amino acid sequence represented by SEQ ID NO: 20. Tenascin C is present in various types of mammals, and a gene encoding tenascin C derived from any of various mammals can be used, however, it is preferred to use a gene of a mammal from which a cell to be used as a host cell is derived. As the tenascin C gene, for example, tenascin C gene of a Chinese hamster comprising a base sequence represented by SEQ ID NO: 8 is exemplified.

The "collagen alpha-1(III) chain gene" means a gene encoding collagen alpha-1(III) chain. Collagen alpha-1(III) chain is a protein comprising an amino acid sequence represented by SEQ ID NO: 21. Collagen alpha-1 (III) is present in various types of mammals, and a gene encoding collagen alpha-1(III) derived from any of various mammals can be used, however, it is preferred to use a gene of a mammal from which a cell to be used as a host cell is derived. As the collagen alpha-1(III) chain gene, for example, collagen alpha-1(III) chain gene of a Chinese hamster comprising a base sequence represented by SEQ ID NO: 10 is exemplified.

The "glutathione S-transferase alpha-3 gene" means a gene encoding glutathione S-transferase alpha-3. Glutathione S-transferase alpha-3 is a protein comprising an amino acid sequence represented by SEQ ID NO: 22. Glutathione S-transferase alpha-3 is present in various types of mammals, and a gene encoding glutathione S-transferase alpha-3 derived from any of various mammals can be used, however, it is preferred to use a gene of a mammal from which a cell to be used as a host cell is derived. As the glutathione S-transferase alpha-3 gene, for example, glutathione S-transferase alpha-3 gene of a Chinese hamster comprising a base sequence represented by SEQ ID NO: 11 is exemplified.

The "calcium/calmodulin-dependent 3',5'-cyclic nucleotide phosphodiesterase 1C gene" means a gene encoding calcium/calmodulin-dependent 3',5'-cyclic nucleotide phosphodiesterase 1C. Calcium/calmodulin-dependent 3',5'-cyclic nucleotide phosphodiesterase 1C is a protein comprising an amino acid sequence represented by SEQ ID NO: 23. Calcium/calmodulin-dependent 3',5'-cyclic nucleotide phosphodiesterase 1C is present in various types of mammals, and a gene encoding calcium/calmodulin-dependent 3',5'-cyclic nucleotide phosphodiesterase 1C derived from any of various mammals can be used, however, it is preferred to use a gene of a mammal from which a cell to be used as a host cell is derived. As the calcium/calmodulin-dependent 3',5'-cyclic nucleotide phosphodiesterase 1C gene, for example, calcium/calmodulin-dependent 3',5'-cyclic nucleotide phosphodiesterase 1C gene of a Chinese hamster comprising a base sequence represented by SEQ ID NO: 12 is exemplified.

The "anthrax toxin receptor 1 gene" means a gene encoding anthrax toxin receptor 1. Anthrax toxin receptor 1 is a protein comprising an amino acid sequence represented by SEQ ID NO: 24. Anthrax toxin receptor 1 is present in various types of mammals, and a gene encoding anthrax toxin receptor 1 derived from any of various mammals can be used, however, it is preferred to use a gene of a mammal from which a cell to be used as a host cell is derived. As the anthrax toxin receptor 1 gene, for example, anthrax toxin receptor 1 gene of a Chinese hamster comprising a base sequence represented by SEQ ID NO: 13 is exemplified.

The "gastrokine-1 gene" means a gene encoding gastrokine-1. Gastrokine-1 is a protein comprising an amino acid sequence represented by SEQ ID NO: 25. Gastrokine-1 is present in various types of mammals, and a gene encoding gastrokine-1 derived from any of various mammals can be used, however, it is preferred to use a gene of a mammal from which a cell to be used as a host cell is derived. As the gastrokine-1 gene, for example, gastrokine-1 gene of a Chinese hamster comprising a base sequence represented by SEQ ID NO: 14 is exemplified.

The "tumor necrosis factor ligand superfamily member 9 gene" means a gene encoding tumor necrosis factor ligand superfamily member 9. Tumor necrosis factor ligand superfamily member 9 is a protein comprising an amino acid sequence represented by SEQ ID NO: 26. Tumor necrosis factor ligand superfamily member 9 is present in various types of mammals, and a gene encoding tumor necrosis factor ligand superfamily member 9 derived from any of various mammals can be used, however, it is preferred to use a gene of a mammal from which a cell to be used as a host cell is derived. As the tumor necrosis factor ligand superfamily member 9 gene, for example, tumor necrosis factor ligand superfamily member 9 gene of a Chinese hamster comprising a base sequence represented by SEQ ID NO: 15 is exemplified.

The "collagen, type VI, $\alpha$2 gene" means a gene encoding collagen, type VI, alpha 2. Collagen, type VI, $\alpha$2 is a protein comprising an amino acid sequence represented by SEQ ID NO: 27. Collagen, type VI, $\alpha$2 is present in various types of mammals, and a gene encoding collagen, type VI, $\alpha$2 derived from any of various mammals can be used, however, it is preferred to use a gene of a mammal from which a cell to be used as a host cell is derived. As the collagen, type VI, $\alpha$2 gene, for example, collagen, type VI, $\alpha$2 gene of a Chinese hamster comprising a base sequence represented by SEQ ID NO: 16 is exemplified.

Among the above-mentioned genes, in the present invention, it is preferred to evaluate the ability of the cell capable of suppressing reduction of a recombinant protein using a difference in the expression level of Pletl [Placenta expressed transcrript 1 protein (ID: 18083239)] gene as an index. Incidentally, in this description, the "ID" denotes a registration number on the web page of Affimetrix, Inc. (https://www.affymetrix.com/analysis/neta6x/xmlquery_ex.affx?netaffx==wtgene_transcript ).

Hereinafter, in this description, the present invention will be described mainly using Pletl gene among the above-mentioned genes, however, the invention is not limited to using Pletl gene, and any of the above-mentioned genes can be used. Further, as listed in Table 2, a gene comprising any one of the base sequences represented by SEQ ID NOS: 2, 3, 5, 6, and 9 whose ID corresponds to 17952793, 17952795, 17957314, 17958207, and 18024962, respectively, can also be used.

In the present invention, the degradation or denaturation of a protein means that the structure of the protein is chemically or enzymatically affected, and a covalent bond or a non-covalent bond is cleaved so that the protein is separated into subunits, protein fragments, polypeptide fragments, amino acids or the like, which constitutes the protein, or that the secondary structure, the tertiary structure, or the quaternary structure of the protein is changed from the naturally occurring state, or that association, aggregation, or dissociation occurs.

In the present invention, the reduction is one of the causes of degradation or denaturation of a protein, and means, for example, that a disulfide bond (-S-S- bond) crosslinking in a molecule or between molecules of the protein is dissociated at a plurality or one or more sites, and a free sulfhydryl group (-SH group) occurs at a plurality or one or more sites.

The reduction of a protein occurs, for example, as follows. An oxidoreductase that is present in a cell is released or the like from the cell and reduces the protein. Examples of the oxidoreductase include dehydrogenases, cytochromes, catalases, oxidases, oxygenases, and fatty acid desaturases. Among these, thioredoxin reductase is considered to be greatly involved in degradation of a protein by reduction.

The present invention can be particularly applied to a protein having a disulfide bond in a molecule or between molecules of the protein. The disulfide bond may be present at one or more sites in a molecule or between molecules of the protein. Further, in the case of an intermolecular disulfide bond, proteins to be bonded may be the same or different.

Examples of the protein having a disulfide bond in a molecule or between molecules include antibodies (for example, IgG1 to 4, IgM, IgE, IgD, and IgA), single-chain antibodies, Fab, and $F(ab')_2$ and the like.

In the present invention, the suppression of reduction or a reducing activity refers to suppression of the above-described reduction of a protein. In particular, in the case of a recombinant protein, it refers that a recombinant protein produced by a cell is maintained in the native form, for example, in a state where a disulfide bond is formed at a proper site.

In the present invention, the ease of reduction of a protein can be determined by homogenizing cells producing a target protein, collecting a solution over time from a cell homogenate incubated under an anaerobic condition, and subsequently, measuring the molecular weight of the produced protein contained in the collected homogenate solution by electrophoresis, capillary electrophoresis, gel filtration HPLC, peptide mapping, or the like. That is, in the case where the produced protein is easily decomposed, the molecular weight of the protein is decreased, and therefore, this is measured by a physicochemical method such as electrophoresis and can be used as an index of the ease of reduction.

Incidentally, even if a protein having a conformation like an antibody is reduced, the conformation is maintained, and therefore, degradation by reduction cannot be detected by an analysis method such as ordinary gel filtration HPLC. In such a case, the reduction can be detected by performing a treatment such as alkylation of a free thiol group formed by reduction so as to denature the protein, and then, performing the above-mentioned analysis.

The cell to be used in the present invention is not particularly limited as long as it is a cell having an ability to express a recombinant protein. Examples thereof include cells derived from mammals that are generally used for producing a recombinant protein such as Chinese hamster ovary cells (CHO), baby hamster kidney cells (BHK), human cells (HT1080 fibrosarcoma cells, Per.C6), mouse myeloma cells (NS0, SP2/0), and Madin-Darby canine kidney cell-derived cells (MDCK), but the cell is not limited thereto, and a cell derived from an animal such as a human, a mouse, a rat, a hamster, a guinea pig, a rabbit, a dog, cattle, a horse, sheep, a monkey, or a pig may be used. Further, as the CHO cells, a substrain such as a CHO-K1 strain, a CHO-DG44 strain, a CHO-S strain, or a DUKX-B11 strain may be used.

In the present invention, the "recombinant protein" means a protein to be obtained by inserting a gene encoding the recombinant protein into an appropriate expression vector using a recombinant DNA technique and transforming a cell using this vector. Examples of the recombinant protein to be used in the present invention include antibodies, and various peptides and proteins having already been used or developed as pharmaceutical products, and cytokines.

As a medium for culturing the cell or a cell obtained by transformation, a medium generally used for culturing a cell as described above or a cell obtained by transformation is used. Examples of such a medium include IMDM, MEM, DMEM, RPMI-1640, X-VIVO15 medium, EX-CELL series media (SAFC Biosciences), BalanCD CHO series media (JX), other commercially available media and custom-made media developed for animal cells.

To the medium, fetal bovine serum may be added, and, a serum-free medium can also be used. When fetal bovine serum is added, the concentration thereof is preferably from about 5 to 20%. Further, in the medium, various components such as amino acids, vitamins, saccharides, soybean hydrolysate, yeast extract, and trace metals can be used by being mixed at an appropriate ratio.

In the first embodiment, in the first step, the expression level of the gene (at least one gene selected from genes comprising any one of the base sequences represented by SEQ ID NOS: 1 to 16 or orthologous genes thereof) in a test cell is measured. The gene whose expression level is measured is preferably Pletl.

A method for measuring the expression level of the gene is not particularly limited, and, for example, a method such as quantitative real-time PCR (qPCR), a reverse transcription quantitative real-time PCR (RT-qPCR) method, an expression difference analysis by RNA-seq using NGS, an expression difference analysis using a DNA microarray, Northern blotting, or ELISA (Enzyme-linked Immunosorbent Assay) is exemplified. Among these, the RT-qPCR method can perform rapid measurement, and therefore is preferred. The RT-qPCR method will be described in detail in the section of Examples.

In the first embodiment, in the second step, the expression level of the gene measured in the first step is (i) compared with a control value of the expression level of the gene in a control cell or (ii) compared with the expression level of the gene in respective cells, and the expression level capable of suppressing reduction of a recombinant protein is evaluated based on a difference therebetween.

The evaluation of the expression level capable of suppressing reduction of a recombinant protein in the second step of the first embodiment is specifically preferably performed according to the following procedures (a) to (d):

(a) calculating a quantitative value by dividing the expression level of the gene in a test cell measured in the first step by the expression level of a standard gene in the test cell,

a relative quantitative value = (the expression level of the gene in a test cell measured in the first step) / (the expression level of a standard gene)

(b) calculating a control value by dividing the expression level of the gene in a control cell by the expression level of the standard gene in the control cell,

a control value = (the expression level of the gene in a control cell) / (the expression level of the standard gene)

(c) calculating a relative expression level by dividing the relative quantitative value for the test cell calculated in (a) by the control value calculated in (b), and

a relative expression level = [the relative quantitative value for the test cell calculated in (a)] / [the control value calculated in (b)]

(d) comparing the relative quantitative value calculated in (a) or the relative expression level calculated in (c) among the respective test cells, and selecting a cell in which each value is high or low.

The procedure (d) is more preferably performed according to the following procedure (d-1) or (d-2):

(d-1) comparing the relative quantitative values calculated in (a) among the respective test cells, and thereby selecting a cell in which the value is high (when at least one gene selected from genes comprising any one of the base sequences represented by SEQ ID NOS: 1 to 9 or orthologous genes thereof is used) or low (when at least one gene selected from genes comprising any one of the base sequences represented by SEQ ID NOS: 10 to 16 or orthologous genes thereof is used), or
(d-2) comparing the relative expression level calculated in (c) among the respective test cells, and thereby selecting a cell in which the value is high (when at least one gene selected from genes comprising any one of the base sequences represented by SEQ ID NOS: 1 to 9 or orthologous genes thereof is used) or low (when at least one gene selected from genes comprising any one of the base sequences represented by SEQ ID NOS: 10 to 16 or orthologous genes thereof is used).

As the cell in which the relative quantitative value is high, a cell in which the relative quantitative value is twice or more, for example, 5 times or more, 7 times or more, 10 times or more, 20 times or more, 25 times or more, 30 times or more, 50 times or more, or 100 times or more the relative quantitative value (also referred to as "control value") of a cell (control cell) showing the smallest value among the test cells is exemplified.

In particular, as the cell in which the relative quantitative value is high, a cell in which the relative quantitative value is preferably 5 times or more, more preferably 10 times or more, further more preferably 20 times or more, still further more preferably 25 times or more, yet still further more preferably 30 times or more, 50 times or more, and most preferably 100 times or more the control value is exemplified.

As the cell in which the relative quantitative value is low, a cell in which the relative quantitative value is 1/2 or less, preferably 1/5 or less, more preferably 1/10 or less, further more preferably 1/20 or less of the relative quantitative value (also referred to as "control value") of a cell (control cell) showing the largest value among the test cells is exemplified.

As the cell in which the relative expression level is high, a cell in which the relative expression level is twice or more, for example, 5 times or more, 7 times or more, 10 times or more, 20 times or more, 25 times or more, 30 times or more, 50 times or more, or 100 times or more the relative expression level of a cell showing the smallest value among the test cells is exemplified.

In particular, as the cell in which the relative expression level is high, a cell in which the relative expression level is preferably 5 times or more, more preferably 10 times or more, further more preferably 20 times or more, still further more preferably 25 times or more, yet still further more preferably 30 times or more, even yet still further more preferably 50 times or more, and most preferably 100 times or more the relative expression level of a cell showing the smallest value among the test cells is exemplified.

As the cell in which the relative expression level is low, a cell in which the relative expression level is 1/2 or less, preferably 1/5 or less, more preferably 1/10 or less, further more preferably 1/20 or less of the relative expression level of a cell showing the largest value among the test cells is exemplified.

Further, the evaluation of the expression level capable of suppressing reduction of a recombinant protein in the second step of the first embodiment can also be performed according to the following procedures (e) to (g):

(e) calculating a relative quantitative value by dividing the expression level of the gene in a test cell measured in the first step by the expression level of a standard gene in the test cell,

$$\text{a relative quantitative value} = (\text{the expression level of the gene in a test cell measured in the first step}) / (\text{the expression level of a standard gene})$$

(f) comparing a relative quantitative value for the test cell calculated in (e) with a relative quantitative value calculated in the same manner for another cell.

(g) comparing the relative quantitative value calculated in (e) among the respective test cells, and thereby selecting a cell in which the value is high (when at least one gene selected from genes comprising any one of the base sequences represented by SEQ ID NOS: 1 to 9 or orthologous genes thereof is used) or low (when at least one gene selected from genes comprising any one of the base sequences represented by SEQ ID NOS: 10 to 16 or orthologous genes thereof is used).

As the standard gene, any gene can be used without particular limitation as long as it is a gene generally expressed in a cell, and examples thereof include GAPDH (Glyceraldehyde-3-phosphate dehydrogenase), β-actin, β2-microglobulin, HPRT1 (Hypoxanthine phosphoribosyltransferase 1), globulin, ubiquitin and the like. Further, the measurement of the expression level of the standard gene can be performed by the RT-qPCR method or the like in the same manner as the expression level of the above-mentioned gene [Genome Biol., 1-11, 3(7), 2002]. Preferably, GAPDH is used.

As the control cell, for example, a cell in which a recombinant protein is easily reduced is exemplified. As the control cell, a sample, which is derived from the same cell as the cell measured for the expression level of the gene in the first step, but whose sampling time is different, may be used, or it may be a cell derived from a different cell. Further, as the control cell, one may be selected from the test cells, and in that case, any cell may be selected among the respective test cells.

Specifically, for example, in the case where the evaluation is performed using at least one gene selected from genes comprising any one of the base sequences represented by SEQ ID NOS: 1 to 9 or orthologous genes thereof, when a cell showing the lowest relative quantitative value among the values of the respective test cells subjected to measurement is used as the control cell, the difference from that of the test cell to be selected becomes larger, and therefore, the analysis of the results is easier.

On the other hand, in the case where the evaluation is performed using at least one gene selected from genes comprising any one of the base sequences represented by SEQ ID NOS: 10 to 16 or orthologous genes thereof, when a cell showing the highest relative quantitative value is used as the control cell, the difference from that of the test cell to be selected becomes larger, and therefore, the analysis of the results is easier.

That is, the control cell is preferably a cell showing the lowest relative quantitative value when at least one gene selected from genes comprising any one of the base sequences represented by SEQ ID NOS: 1 to 9 or orthologous genes thereof is used, and is a cell showing the highest relative quantitative value when at least one gene selected from genes comprising any one of the base sequences represented by SEQ ID NOS: 10 to 16 or orthologous genes thereof is used.

A second embodiment of the present invention is a method for selecting a cell in which a reducing activity for a recombinant protein is suppressed, comprising the following first step and second step:

a first step: measuring the expression level of a protein encoded by at least one gene selected from genes comprising any one of the base sequences represented by SEQ ID NOS: 1 to 16 or orthologous genes thereof; and

a second step: comparing the expression level of the protein encoded by the gene measured in the first step with a control value of the expression level of the protein encoded by the gene in a control cell or comparing the expression level of the protein encoded by the gene in the respective cells, and evaluating the expression level capable of suppressing reduction of a recombinant protein based on a difference therebetween.

In the second embodiment, in the first step, the expression level of a protein encoded by the gene (at least one gene selected from genes comprising any one of the base sequences represented by SEQ ID NOS: 1 to 16 or orthologous genes thereof) expressed in a test cell is measured. The protein whose expression level is measured is preferably Pletl.

A method for measuring the expression level of the protein is not particularly limited, and, for example, a method such as ELISA, Western blotting, FACS (Fluorescence-activated Cell Sorting), HPLC (High Performance Liquid Chromatography), or LC-MS is exemplified. Among these, the ELISA method can perform rapid measurement, and therefore is preferred.

In the second embodiment, in the second step, the expression level of a protein encoded by the gene (at least one gene selected from genes comprising any one of the base sequences represented by SEQ ID NOS: 1 to 16 or orthologous genes thereof) measured in the first step is (i) compared with a control value of the expression level of a protein encoded by the gene in a control cell or (ii) compared with the expression level of a protein encoded by the gene in respective cells, and the expression level capable of suppressing reduction of a recombinant protein is evaluated based on a difference therebetween.

The evaluation in the second step of the second embodiment is specifically preferably performed according to the following procedures (a) to (d):

(a) calculating a relative quantitative value by dividing the expression level of a protein encoded by the gene in a test cell measured in the first step by the expression level of a protein (standard protein) encoded by a standard gene in the test cell,

a relative quantitative value = (the expression level of a protein encoded by the gene in a test cell measured in the first step) / (the expression level of a standard protein)

(b) calculating a control value by dividing the expression level of a protein encoded by the gene in a control cell by the expression level of the standard protein in the control cell,

a control value = (the expression level of a protein encoded by the gene in a control cell) / (the expression level of the standard protein)

(c) calculating a relative expression level by dividing the relative quantitative value for the test cell calculated in (a) by the control value calculated in (b),

a relative expression level = [the relative quantitative value for the test cell calculated in (a)] / [the control value calculated in (b)]

(d) comparing the relative quantitative value calculated in (a) or the relative expression level calculated in (c) among the respective test cells, and selecting a cell in which each value is high or low.

The procedure (d) is more preferably performed according to the following procedure (d-1) or (d-2):

(d-1) comparing the relative quantitative value calculated in (a) among the respective test cells, and thereby selecting a cell in which the value is high (when a protein encoded by at least one gene selected from genes comprising any one of the base sequences represented by SEQ ID NOS: 1 to 9 or orthologous genes thereof is used) or low (when a protein encoded by at least one gene selected from genes comprising any one of the base sequences represented by SEQ ID NOS: 10 to 16 or orthologous genes thereof is used), or

(d-2) comparing the relative expression level calculated in (c) among the respective test cells, and thereby selecting a cell in which the value is high (when a protein encoded by at least one gene selected from genes comprising any one of the base sequences represented by SEQ ID NOS: 1 to 9 or orthologous genes thereof is used) or low (when a protein encoded by at least one gene selected from genes comprising any one of the base sequences represented by SEQ ID NOS: 10 to 16 or orthologous genes thereof is used).

As the cell in which the relative quantitative value is high, a cell in which the relative quantitative value is twice or more, for example, 5 times or more, 7 times or more, 10 times or more, 20 times or more, 25 times or more, 30 times or more, 50 times or more, or 100 times or more the relative quantitative value (also referred to as "control value") of a cell (control cell) showing the smallest value among the test cells is exemplified.

In particular, as the cell in which the relative quantitative value is high, a cell in which the relative quantitative value is preferably 5 times or more, more preferably 10 times or more, further more preferably 20 times or more, still further more preferably 25 times or more, yet still further more preferably 30 times or more, even yet still further more preferably 50 times or more, and most preferably 100 times or more the control value is exemplified.

As the cell in which the relative quantitative value is low, a cell in which the relative quantitative value is 1/2 or less, preferably 1/5 or less, more preferably 1/10 or less, further more preferably 1/20 or less of the relative quantitative value (also referred to as "control value") of a cell (control cell) showing the largest value among the test cells is exemplified.

As the cell in which the relative expression level is high, a cell in which the relative expression level is twice or more, for example, 5 times or more, 7 times or more, 10 times or more, 20 times or more, 25 times or more, 30 times or more, 50 times or more, or 100 times or more the relative expression level of a cell showing the smallest value among the test cells is exemplified.

In particular, as the cell in which the relative expression level is high, a cell in which the relative expression level is preferably 5 times or more, more preferably 10 times or more, further more preferably 20 times or more, still further more preferably 25 times or more, yet still further more preferably 30 times or more, even yet still further more preferably 50 times or more, and most preferably 100 times or more the relative expression level of a cell showing the smallest value among the test cells is exemplified.

As the cell in which the relative expression level is low, a cell in which the relative expression level is 1/2 or less, preferably 1/5 or less, more preferably 1/10 or less, further more preferably 1/20 or less of the relative expression level of a cell showing the largest value among the test cells is exemplified.

Further, the evaluation of the expression level capable of suppressing reduction of a recombinant protein in the second step of the second embodiment can also be performed according to the following procedures (e) to (g):

(e) calculating relative quantitative value obtained by dividing the expression level of a protein encoded by the gene in a test cell measured in the first step by the expression level of a protein (standard protein) encoded by a standard gene in the test cell,

$$\text{a relative quantitative value} = (\text{the expression level of a protein encoded by the gene in a test cell measured in the first step}) / (\text{the expression level of a standard protein})$$

(f) comparing the relative quantitative value for the test cell calculated in (e) with a relative quantitative value calculated in the same manner for another cell, and

(g) comparing the relative quantitative value calculated in (e) among the respective test cells, and thereby selecting a cell in which the value is high (when genes represented by SEQ ID NOS: 1 to 9 are used) or low (when genes represented by SEQ ID NOS: 10 to 16 are used) is selected.

As the standard protein, any protein can be used without particular limitation as long as it is a protein generally expressed in a cell, and examples thereof include GAPDH (Glyceraldehyde-3-phosphate dehydrogenase), β-actin, β2-microglobulin, HPRT1 (Hypoxanthine phosphoribosyltransferase 1), globulin, ubiquitin and the like.

Further, the measurement of the expression level of the standard protein can be performed by the ELISA method or the like in the same manner as the expression level of a protein encoded by the above-mentioned gene. Preferably, GAPDH is used.

As the control cell, for example, a cell in which a recombinant protein is easily reduced is exemplified. As the control cell, a sample, which is derived from the same cell as the cell measured for the expression level of a protein encoded by the gene in the first step, but whose sampling time is different, may be used, or it may be a cell derived from a different cell. Further, as the control cell, one may be selected from the test cells, and in that case, any cell may be selected among the respective test cells.

Specifically, for example, in the case where the evaluation is performed using a protein encoded by at least one gene selected from genes comprising any one of the base sequences represented by SEQ ID NOS: 1 to 9 or orthologous genes thereof, when a cell showing the lowest relative quantitative value among the values of the respective test cells subjected to measurement is used as the control cell, the difference from that of the test cell to be selected becomes larger, and therefore, the analysis of the results is easier.

On the other hand, in the case where the evaluation is performed using a protein encoded by at least one gene selected from genes comprising any one of the base sequences represented by SEQ ID NOS: 10 to 16 or orthologous genes thereof, when a cell showing the highest relative quantitative value is used as the control cell, the difference from that of the test cell to be selected becomes larger, and therefore, the analysis of the results is easier.

That is, the control cell is preferably a cell showing the lowest relative quantitative value when a protein encoded by at least one gene selected from genes comprising any one of the base sequences represented by SEQ ID NOS: 1 to 9 or orthologous genes thereof is used, and is a cell showing the highest relative quantitative value when a protein encoded by at least one gene selected from genes comprising any one of the base sequences represented by SEQ ID NOS: 10 to 16 or orthologous genes thereof is used.

In the first embodiment or the second embodiment, a step of introducing at least one gene selected from genes comprising any one of the base sequences represented by SEQ ID NOS: 1 to 9 or orthologous genes thereof into a cell, thereby transforming the cell may be included before the first step.

In the present invention, the "introducing a gene" means that the target gene is present in the cell. For example, the target gene is present in the chromosome of the cell or the target gene is included in a vector present in the cell. As the target gene, a gene of a recombinant protein to be produced by a cell and/or at least one gene selected from genes comprising any one of the base sequences represented by SEQ ID NOS: 1 to 9 or orthologous genes thereof is exemplified.

The first embodiment or the second embodiment may comprise, before the first step, a step of introducing at least one gene selected from genes comprising any one of the base sequences represented by SEQ ID NOS: 1 to 9 or orthologous genes thereof into a cell, thereby transforming the cell, and a step of introducing a gene encoding a recombinant protein into a cell, thereby transforming the cell. Either of these steps may be performed first or these steps may be performed simultaneously. As a method for introducing the gene to transform the cell, the above-mentioned method is exemplified.

In the introduction of the target gene into a cell, for example, a known method such as an electroporation method, a calcium phosphate method, a liposome method, or a DEAE dextran method can be used. When a CHO cell is used as a host cell, for example, the target gene is introduced into an expression vector, and the resulting vector is transfected into the CHO cell by a lipofection method or the like, and the resulting cell is cultured, thereby the CHO cell transfected with the gene can be obtained.

As a method for introducing the target gene into a cell, an appropriate method can be used according to the cell, and for example, a method using an expression vector is exemplified. The expression vector is not particularly limited, and a plasmid vector is preferably used. Other than this, for example, a viral vector, a cosmid vector, a bacterial artificial chromosome (BAC), a yeast artificial chromosome (YAC), and other non-plasmid vectors may be used. As these vectors, commercially available vectors can also be used. Incidentally, when a cell derived from a mammal is used as the cell, as the expression vector, an expression vector comprising a promoter, a splicing region, a poly-A addition site, etc. is preferably used.

Further, in the first embodiment or the second embodiment, a step of knocking down or knocking out at least one gene selected from genes comprising any one of the base sequences represented by SEQ ID NOS: 10 to 16 or orthologous genes thereof in the cell, thereby downregulating the expression level of the gene may be included before the first step.

In the present invention, the "knocking down or knocking out" means that transcription or translation of the target gene is inhibited and reduced as compared with normal cells. By doing this, the function of the target gene is lost or attenuated.

In the knocking down or knocking out of the gene, any technique can be used as long as the function of the gene is suppressed or lost. For example, the expression level of the gene can be suppressed using a method for introducing an antisense RNA into a cell, an RNAi method using an siRNA, a microRNA, or the like, etc. Further, it is also possible to use a method for knocking out the gene using a genome-editing technique such as TALEN or CRISPR-Cas9.

The first embodiment or the second embodiment may comprise, before the first step, a step of knocking down or knocking out at least one gene selected from genes comprising any one of the base sequences represented by SEQ ID NOS: 10 to 16 or orthologous genes thereof in a cell, and a step of introducing a gene encoding a recombinant protein into a cell, thereby transforming the cell. Either of these steps may be performed first or these steps may be performed simultaneously. As a method for knocking down or knocking out the gene and a method for introducing the gene to transform the cell, the above-mentioned methods are exemplified.

There is a high possibility that the thus selected cell has an ability to suppress reduction of a target recombinant protein, and by using the cell as a host cell, a cell producing any recombinant protein can be obtained. The recombinant protein produced by the cell (production cell) obtained by the selection method of the present invention can be isolated without being degraded or denatured by reduction.

When a protein-producing cell is established, it is often the case that several tens to several thousands or more of cell populations are established, and an appropriate cell for producing a recombinant protein is selected therefrom. The production cell obtained based on the method of the present invention has a property of suppressing reduction of a recombinant protein. At least the probability that the production cell obtained based on the method of the present invention has a property of suppressing reduction of a recombinant protein is high. That is, according to the present invention, the probability of acquisition of a cell capable of suppressing reduction of a recombinant protein can be increased as compared with a conventional method.

The selection method of the present invention can be combined with a method for selecting a production cell based on another index such as productivity, high expression, or quality. Therefore, the present invention can provide a method for acquiring a cell highly producing or highly expressing a recombinant protein.

A third embodiment of the present invention is a cell capable of suppressing reduction of a recombinant protein, and acquisition thereof including introducing at least one gene selected from genes comprising any one of the base sequences represented by SEQ ID NOS: 1 to 9 or orthologous genes thereof into a cell, thereby transforming the cell.

A fourth embodiment of the present invention is a cell capable of suppressing reduction of a recombinant protein, in which at least one gene selected from genes comprising any one of the base sequences represented by SEQ ID NOS: 10 to 16 or orthologous genes thereof is knocked down or knocked out, and acquisition thereof.

By using a cell into which at least one gene selected from genes comprising any one of the base sequences represented by SEQ ID NOS: 1 to 9 or orthologous genes thereof is introduced, or a cell in which at least one gene selected from genes comprising any one of the base sequences represented by SEQ ID NOS: 10 to 16 or orthologous genes thereof is knocked down or knocked out as a host cell, reduction of a recombinant protein produced from the cell and degradation or denaturation accompanying this can be suppressed. Accordingly, a production cell in which decrease or loss of the activity of a recombinant protein is suppressed can be obtained.

By culturing the cell obtained according to the present invention in an ordinary manner, a target protein can be produced in the native form, that is, a form in which a disulfide bond is formed at a proper site. As the method for culturing the cell, a well known method for producing a protein such as batch culture, fed-batch culture, or perfusion culture can be used. The obtained culture broth is subjected to purification after being subjected to a cell separation step. The cell separation step is performed by a method such as continuous centrifugation, batch centrifugation, filtration, or perfusion.

In order to isolate and purify the recombinant protein from the cell, known separation operations can be performed in combination. Specifically, for example, a treatment with a denaturing agent such as urea or a surfactant, a sonication treatment, enzymatic digestion, salt precipitation, a solvent fractional precipitation method, dialysis, centrifugation, ultrafiltration, gel filtration, SDS-PAGE, isoelectric focusing, ion exchange chromatography, hydrophobic chromatography, affinity chromatography, reverse-phase chromatography, and the like are exemplified, however, the method is not limited thereto.

Examples

Next, the present invention will be described in more detail with reference to Examples, however, the invention is by no means limited thereto.

[Example 1]

Gene Expression Difference Analysis of Antibody-Producing Cell Using Microarray Analysis

A gene expression difference analysis using a DNA microarray was performed for the purpose of specifying a gene (mRNA) expressed correlatively with a difference in ease of reduction of a protein (antibody) to be produced.

Cells in which an antibody to be produced is not easily reduced [a CHO cell #1 producing an IgG4-type monoclonal antibody (hereinafter referred to as "antibody A") (hereinafter abbreviated as "A#1 strain"), and a CHO cell #1 producing an IgG1/IgG3 chimeric monoclonal antibody (hereinafter referred to as "antibody B") (hereinafter abbreviated as "B#1 strain")] that is an antibody against an antigen different from the antibody A, and cells in which an antibody to be produced is easily reduced [a CHO cell #2 producing the antibody A (hereinafter abbreviated as "A#2 strain"), and a CHO cell #2 producing the antibody B (hereinafter abbreviated as "B#2 strain")] were used for evaluation. The level of ease of reduction in each cell is shown in Table 1. Incidentally, evaluation of the ease of reduction was performed as follows.

That is, cells were inoculated in a 250-mL Erlenmeyer flask and cultured for 13 days in a $CO_2$ incubator. During the culture period, a feed medium was appropriately added, and also sampling was performed. A viable cell density and a viability were measured using a live/dead cell autoanalyzer (Vi-CELL XR), and the concentration of the antibody was measured using Protein A HPLC.

The cells on day 13 of culture were homogenized using an ultrasonic homogenizer (VP-300, manufactured by TAITEC Corporation), and a cell homogenate was incubated under an anaerobic condition. Sampling was performed from the solution over time, and capillary electrophoresis was performed using Agilent 2100 Bioanalyzer (manufactured by Agilent Co., Ltd.), and the degradation state of the produced protein was evaluated. The results for the A#1 strain and the A#2 strain are shown in FIG. 1.

As shown in FIG. 1, when changes in the structures of the antibodies produced by the A#1 strain and the A#2 strain were observed, it was confirmed that in the case of the A#1 strain, the band of the target product (Whole antibody, at around 150 kDa) did not change, however, in the case of the A#2 strain, the degradation products having a low molecular weight (28 to 95 kDa) were increased over time with the progress of the reduction reaction.

[Table 1]

| Production cell | Ease of reduction |
|---|---|
| A#1 | low |
| A#2 | high |
| B#1 | low |
| B#2 | high |

These cells were cultured for 14 days in a $CO_2$ incubator using a 250-mL Erlenmeyer flask. During the culture period, addition of a feed medium and sampling were appropriately performed. On day 3 and day 9 of culture, sampling was performed from each cell culture broth, and total RNA was extracted from about $1 \times 10^7$ cells of each using ISOGEN (manufactured by Nippon Gene Co., Ltd.).

By using a 50 $\mu$g portion of the obtained total RNA, a DNase treatment was performed, and after a phenol-chloroform treatment and ethanol precipitation were performed, a microarray analysis (manufactured by Takara Bio, Inc.) was performed from a solution containing the total RNA in an amount of 1 $\mu$g or more for each using GeneChip CHO Gene 2.0 ST Array (manufactured by Affymetrix, Inc.).

Data analysis was performed using GeneSpring GX (manufactured by Agilent Co., Ltd.), and a numerical value correction method was performed using the RMA method or the PLIER method (Yi Qu et al. BMC Bioinformatics, 211, 11, 2010).

By the analysis results, 29700 genes whose expression levels are different were confirmed. From the data created by each correction method, 16 genes whose expression level is different twice or more between the strain in which the antibody is not easily reduced and the strain in which the antibody is easily reduced, were specified. The list of the 16 genes are shown in Table 2.

[Table 2]

| Transcript Cluster Sequence ID | NCBI Reference Sequence ID | Description of Gene | Ratio of Expression Level (cell strain with low susceptibility to reduction/cell with high susceptibility to reduction) |
|---|---|---|---|
| 18083239 | XM_ 003498539.3: 95-718 | placenta-expressed transcript 1 protein | High expression, twice or more |
| 17952793 | | uncharacterized | |
| 17952795 | | uncharacterized | |
| 17952194 | XM_ 003504576.3: 16-1887 | matrilin 4 (Matn4) | |
| 17957314 | | uncharacterized | |
| 17958207 | | uncharacterized | |
| 17968846 | XM_ 007653651.2: 513-3224 | G-protein-coupled receptor 133 | |
| 18020489 | XM_ 003506418.3: 373-6432 | tenascin C | |
| 18024962 | | uncharacterized | |

(continued)

| Transcript Cluster Sequence ID | NCBI Reference Sequence ID | Description of Gene | Ratio of Expression Level (cell strain with low susceptibility to reduction/cell with high susceptibility to reduction) |
|---|---|---|---|
| 17955459 | XM_ 003515418.2: 264-4664 | collagen alpha-1(III) chain | Low expression, half or less |
| 17967811 | XM_ 003513514.3: 198-863 | glutathione S-transferase alpha-3 | |
| 18038891 | XM_ 003503929.3: 51-1874 | calcium/calmodulin-dependent 3', 5'-cyclic nucleotide phosphodiesterase 1C | |
| 18042016 and 18113632 | XM_ 007644176.2: 386-1984 | anthrax toxin receptor 1 | |
| 18042016 and 18113632 | XM_ 003503574.1 | gastrokine-1 | |
| 18056819 | XM_ 003501810.3 | tumor necrosis factor ligand superfamily member 9 | |
| 18066454 | XM_ 003500554.3: 188-3268 | collagen, type VI, alpha 2 | |

It is considered that by using the expression level of the specified gene shown in Table 2 or the production amount of the protein encoded thereby as an index, a cell in which a reducing activity for a target protein such as an antibody is different can be specified.

[Example 2]

Determination of Ease of Reduction of Production Cell by Expression Difference Analysis of Placenta-Expressed Transcript 1 Protein (hereinafter referred to as Pletl) Gene Using RT-qPCR Method

The following analysis was performed using Pletl gene that was revealed to be highly expressed in a production strain in which a protein to be produced is not easily reduced among the genes found in Example 1 as an example.

With respect to the total RNA obtained from four types of cells (Table 1) prepared in Example 1 and used in the microarray analysis, the expression level of a gene was analyzed by the following method using the RT-qPCR method. First, based on the information of the base sequence of Pletl gene of a Chinese hamster, a probe and primers to be used in the RT-qPCR method were designed (Table 3, SEQ ID NOS: 3 to 5).

A reaction solution was prepared as 20 $\mu$L of a reaction system containing 5 $\mu$L of TaqMan Fast Virus 1-Step Master Mix (manufactured by Thermo Fisher Scientific, Inc.) and using the probe at 200 nM (final concentration), the primers at 500 nM (final concentration), and 10 ng of the total RNA per reaction. The reaction was performed using a real-time PCR device (7900HT Fast Real Time PCR System, manufactured by Applied Biosystems, Inc.) according to a schedule in which after a pre-reaction at 50°C for 5 minutes $\rightarrow$ 95°C for 20 seconds, a cycle of "95°C for 3 seconds $\rightarrow$ 60°C for 30 seconds" is repeated 40 times.

The gene expression level of Pletl was compared among cells using a relative expression level. Specifically, first, a relative quantitative value corrected by dividing the gene expression level of Pletl by the gene expression level of GAPDH was calculated. Subsequently, a cell showing the lowest relative quantitative value was used as a control cell, and a relative expression level was calculated by dividing the relative quantitative value of each of the other cells by the relative quantitative value (control value) of the control cell. Incidentally, GAPDH used here is one example of a housekeeping gene generally expressed in a cell. The evaluation results are shown in Table 4.

[Table 3]

| Name | Base Sequence |
|---|---|
| Probe (obtained by attaching FAM to the 5' end of SEQ ID NO: 28 and attaching MGB to the 3' end thereof) | 5'-FAM-caatgacagcagtgtcct-MGB-3' |
| Forward Primer (SEQ ID NO: 28) | 5'-gacccctgcatggtctttga-3' |
| Reverse Primer (SEQ ID NO: 29) | 5'-accgtaatgctgactcccaagt-3' |

[Table 4]

| Sampling day | Relative expression level | |
|---|---|---|
| Day 3 | A#1 strain | 97 |
| | A#2 strain | 1 |
| | B#1 strain | 208 |
| | B#2 strain | 1 |
| Day 9 | A#1 strain | 84 |
| | A#2 strain | 1 |
| | B#1 strain | 52 |
| | B#2 strain | 1 |

As shown in Table 4, it was confirmed that in the cells in which the antibody is not easily reduced (A#1 strain and B#1 strain), the relative expression level of Pletl gene is 52 to 208 times higher than in the cells in which the antibody is easily reduced (A#2 strain and B#2 strain).

From this result, it is considered that by quantitatively determining the expression level of mRNA of Pletl gene using a quantitative gene analysis method such as the RT-qPCR method, a production cell in which a target protein is not easily reduced can be easily specified.

[Example 3]

Selection of Production Cell in which Protein is Not Easily Reduced by Expression Difference Analysis of Pletl Gene

Chinese hamster ovary-derived cells (CHO cells) were acclimated in a serum-free medium, whereby host cells X were obtained. By using the method described in US Patent No. 6946292, CHO cells in which fucosyltransferase (FUT8) was deleted were obtained and used as host cells Y.

Subsequently, the host cells Y were suspended in PBS, and gene transfer was performed using an expression vector integrated with a gene of an IgG1-type monoclonal antibody (hereinafter referred to as "antibody C") that is an antibody against an antigen different from the antibody A or the antibody B.

After gene transfer by electroporation, the cells in a cuvette were suspended in a medium and inoculated into a 96-well plate and cultured in a $CO_2$ incubator for a few days. Subsequently, after a few days from the gene transfer, the medium was exchanged with a medium containing cycloheximide and the cells were cultured for 4 weeks while performing medium exchange once a week. During that period, the size of the plate was appropriately changed, and the cells were grown to a scale of a 125-mL volume Erlenmeyer flask in the end. The culture supernatant was sampled, and cells which produced the antibody in a large amount were selected and subjected to single-cell cloning.

Subsequently, the obtained cells were inoculated into a 384-well plate using a flow cytometer (FACS Aria II, manufactured by Becton, Dickinson and Company) or a limiting dilution method. Culture was continued, and cells were obtained from a well in which a single colony was formed, and the cells were subjected to extended culture to a scale of a 125-mL volume Erlenmeyer flask.

From these cells, cells were selected using the productivity of the antibody as an index, and 46 types of cells were obtained, whereby 46 strains of antibody C-producing cells were prepared. The 46 strains of antibody C-producing cells were named antibody C-producing strain #1 to #46, respectively. The cells of each strain were dispensed in a 1-mL vial and cryopreserved, respectively.

Subsequently, the respective cells were cultured for 14 days in a CO2 incubator using a 250-mL volume Erlenmeyer flask. During the culture period, a feed medium was appropriately added, and also sampling was performed. On day 3 of culture, sampling was performed from each cell culture broth (1 mL each), and a cell pellet (about $3\times10^6$

cells) recovered by centrifugation was dissolved by adding 500 μL of ISOGEN, whereby total RNA was obtained.

Subsequently, by using this, the gene expression level of Pletl was measured by the RT-qPCR method in the same manner as in Example 2. At that time, GAPDH was selected as one example of a standard gene, and a relative quantitative value of each antibody C-producing strain with respect to the expression level of GAPDH was calculated. Further, as one example, the antibody C-producing strain #5 was used as a control cell. By assuming the relative quantitative value of Pletl of the antibody C-producing strain #5 to be 1.0, the relative expression level of Pletl of each cell was calculated. Further, on day 14, the ease of reduction of the antibody to be produced was evaluated for each cell in the same manner as in Example 1. The results are shown in Table 5.

[Table 5]

| Antibody C-producing strain | Relative expression level | Whether antibody was reduced or not |
|---|---|---|
| #1 | 14.9 | reduced |
| #2 | 2.0 | reduced |
| #3 | 2.6 | reduced |
| #4 | 1.4 | reduced |
| #5 | 1.0 | reduced |
| #6 | 2.5 | reduced |
| #7 | 2.6 | reduced |
| #8 | 1.6 | reduced |
| #9 | 1.5 | reduced |
| #10 | 2.3 | reduced |
| #11 | 675.6 | not reduced |
| #12 | 955.4 | not reduced |
| #13 | 3.2 | reduced |
| #14 | 2.8 | reduced |
| #15 | 5.3 | reduced |
| #16 | 1.9 | reduced |
| #17 | 2.8 | reduced |
| #18 | 10.6 | reduced |
| #19 | 3.0 | reduced |
| #20 | 8.0 | reduced |
| #21 | 36.8 | not reduced |
| #22 | 724.1 | reduced |
| #23 | 36.8 | not reduced |
| #24 | 18.4 | reduced |
| #25 | 9.8 | reduced |
| #26 | 1.6 | reduced |
| #27 | 1.7 | reduced |
| #28 | 1.2 | reduced |
| #29 | 1.6 | reduced |
| #30 | 2.0 | reduced |
| #31 | 2.6 | reduced |
| #32 | 4.0 | reduced |
| #33 | 8.6 | reduced |
| #34 | 1.7 | reduced |
| #35 | 2.0 | reduced |
| #36 | 2.6 | reduced |
| #37 | 0.0 | reduced |
| #38 | 4.3 | not reduced |
| #39 | 1.0 | not reduced |
| #40 | 4.9 | reduced |
| #41 | 7.5 | reduced |

(continued)

| Antibody C-producing strain | Relative expression level | Whether antibody was reduced or not |
|---|---|---|
| #42 | 388.0 | not reduced |
| #43 | 222.9 | not reduced |
| #44 | 21.1 | not reduced |
| #45 | 48.5 | not reduced |
| #46 | 362.0 | not reduced |

There were 11 strains (24%) of cells in which the antibody was not reduced (#11, #12, #21, #23, #38, #39, #42, #43, #44, #45, and #46) among the 46 cells of the antibody C-producing strains. On the other hand, there existed 18 production strains in which the relative expression level of Pletl is 5 times or more higher than that of the antibody C-producing strain #5 (#1, #11, #12, #15, #18, #20 to #25, #33, and #41 to #46), and among these, there existed 10 production strains (56%) in which the antibody was not reduced (#11, #12, #21, #23, and #41 to #46). There existed 13 strains of production cells in which the relative expression level of Pletl is 10 times or more higher than that of the antibody C-producing strain #5 (#1, #11, #12, #18, #21 to #24, and #42 to #46), and among these, there existed 9 strains (69%) of cells in which the antibody was not reduced.

Further, there existed 11 strains of production cells in which the relative expression level of Pletl is 15 times or more higher than that of the antibody C-producing strain #5, and among these, there existed 9 strains (82%) of cells in which the antibody was not reduced (#11, #12, #21, #23, and #42 to #46). In addition, there existed 6 strains of production cells in which the relative expression level of Pletl is 100 times or more higher than that of the antibody C-producing strain #5 (#11, #12, #22, #42, #43, and #46), and among these, there existed 5 strains (83%) of cells in which the antibody was not reduced (#11, #12, #42, #43, and #46).

From this result, it was confirmed that by selecting a cell in which the expression level of Pletl gene is high using the RT-qPCR method, a cell in which a target protein is not reduced can be obtained with high probability. Incidentally, it was also shown that by arbitrarily increasing the relative expression level of Pletl gene, for example, from 5 times to 100 times as compared with the control value, the probability that a target protein is not reduced can be increased.

[Example 4]

Confirmation of Versatility of Selection of Production Cell in which Target Protein is Not Easily Reduced by Expression Difference Analysis of Pletl Gene

For the purpose of confirming the versatility of the method for specifying a production strain in which a target protein is not easily reduced by an expression difference analysis of Pletl gene, an examination was performed using antibody D-producing cells.

According to the method shown in Example 3, a gene of an IgG4-type monoclonal antibody (hereinafter referred to as "antibody D") that is an antibody against an antigen different from the antibody A, the antibody B, or the antibody C was introduced into CHO cells, followed by single-cell cloning, whereby 6 strains of antibody D-producing cells were prepared and named antibody D-producing strain #1 to #6, respectively.

The respective cells were cultured for 14 days in a $CO_2$ incubator using a 250-mL volume Erlenmeyer flask. During the culture period, a feed medium was appropriately added, and also sampling was performed. On day 14 of culture, the ease of reduction of the antibody to be produced was evaluated in the completely same manner as in Example 1. Subsequently, a cryopreserved stock prepared during subculture of each cell was thawed, and a cell pellet (about $3\times10^6$ cells) recovered by centrifugation was dissolved by adding 500 $\mu$L of ISOGEN, whereby total RNA was obtained.

By using this, the gene expression level of Pletl was measured by the RT-qPCR method in the same manner as in Example 2. GAPDH was selected as one example of a standard gene, and a relative quantitative value corrected by dividing the gene expression level of Pletl by the gene expression level of GAPDH was calculated. Further, one cell (the antibody D-producing strain #5) was selected as a control cell, and by using the relative quantitative value of the strain (the antibody D-producing strain #5) as a control value, the relative expression level of Pletl of each of the other cells was calculated. The results of the gene expression level of Pletl in the antibody D-producing strains and evaluation of reduction of the antibody were compared. The results are shown in Table 6.

[Table 6]

| Antibody D-producing strain | Relative expression level | Whether antibody was reduced or not |
|---|---|---|
| #1 | 24.9 | not reduced |

(continued)

| Antibody D-producing strain | Relative expression level | Whether antibody was reduced or not |
|---|---|---|
| #2 | 42.5 | not reduced |
| #3 | 1.2 | reduced |
| #4 | 70.8 | reduced |
| #5 | 1.0 | reduced |
| #6 | 7.7 | not reduced |

As shown in Table 6, there were 3 strains (50%) of cells in which the antibody was not reduced (#1, #2, and #6) among the 6 antibody D-producing strains. There existed 4 strains of production cells in which the relative expression level of Pletl gene is 5 times or more higher than that of the antibody D-producing strain #5 (#1, #2, #4, and #6), and among these, there existed 3 strains (75%) of cells in which the antibody was not reduced (#1, #2, and #6). As described above, it was confirmed that by analyzing the gene expression level of Pletl, a production strain in which reduction does not easily occur can be obtained regardless of the type of the antibody or the production strain.

[Example 5]

Expression Analysis of Pletl Gene using Cell during Subculture

Among the 46 cells of the antibody C-producing strains for which evaluation was performed in Example 3, 9 cells in which the gene expression level of Pletl is different were subcultured for 80 days, and the relative expression level of Pletl gene was analyzed. The results are shown in FIG. 2.

As shown in FIG. 2, in the 80 days of subculture period, the expression level of Pletl gene was stable. As described above, even if the number of generation of the cells was changed, the expression level of Pletl gene was constant, and therefore, it was confirmed that a cell in which the ease of reduction is different can be selected using Pletl as an index without depending on the state of the cell such as the number of generations.

[Example 6]

Acquisition of Host Cells (CHO cells X' and Y') in which Recombinant Protein is Not Easily Reduced

By using the two types of host cells (X and Y) obtained in Example 3, single-cell cloning was performed by the following method, whereby subcloning strains were produced.

Each cell (X or Y) was subjected to extended culture to a scale of a 125-mL Erlenmeyer flask. Subsequently, by using Vi-CELL, a viable cell density was measured, and a cell solution was prepared so as to contain about 16 cells per 100 well. The cell solution was inoculated into a 384-well plate in an amount of 50 μL each, and the cells were cultured. The cells in a well in which a single colony was formed were cultured and grown to a scale of a 125-mL volume Erlenmeyer flask, and thereafter, the cells were dispensed in an amount of 1 mL each and cryopreserved.

By using the cells obtained during culture in the above-mentioned process, an expression analysis of Pletl gene was performed by the RT-qPCR method in the same manner as in Example 2. With respect to 40 strains of each of the subcloning strains of the host cells X and Y, a strain showing the lowest gene expression level of Pletl was used as a control cell, and the relative expression level was calculated in the same manner as the above-mentioned Examples.

16 strains in which the gene expression level of Pletl is high were obtained for each (X' strain and Y' strain). Further, as comparison subjects, two strains for each (Xc strains and Yc strains) in which the relative expression level of Pletl is low were obtained as control cells. The results are shown in Table 7.

[Table 7]

| Host cell | Relative expression level | Host cell | Relative expression level |
|---|---|---|---|
| X'#1 | 22 | Y'#1 | 2 |
| X'#2 | 52 | Y'#2 | 18 |
| X'#3 | 21 | Y'#3 | 5 |
| X'#4 | 21 | Y'#4 | 9 |

(continued)

| Host cell | Relative expression level | Host cell | Relative expression level |
|---|---|---|---|
| X'#5 | 111 | Y'#5 | 20 |
| X'#6 | 110 | Y'#6 | 31 |
| X'#7 | 84 | Y'#7 | 12 |
| X'#8 | 23 | Y'#8 | 20 |
| X'#9 | 36 | Y'#9 | 6 |
| X'#10 | 17 | Y'#10 | 65 |
| X'#11 | 28 | Y'#11 | 13 |
| X'#12 | 45 | Y'#12 | 17 |
| X'#13 | 92 | Y'#13 | 24 |
| X'#14 | 56 | Y'#14 | 5 |
| X'#15 | 57 | Y'#15 | 13 |
| X'#16 | 22 | Y'#16 | 15 |
| Xc#1 (standard strain) | 1 | Yc#1 (standard strain) | 1 |
| Xc#2 | 4 | Yc#2 | 1 |

As shown in Table 7, in the case of the X' strain, there existed 9 strains in which the relative expression level of Pletl gene is 30 times or more higher than that of the Xc#1 strain (X'#2, X'#5, X'#6, X'#7, X'#9, and X'#12 to X'#15), and there existed 7 strains in which the relative expression level of Pletl gene is 50 times or more higher than that of the Xc#1 strain (X'#2, X'#5, X'#6, X'#7, and X'#13 to X'#15). In the case of the Y' strain, there existed 11 strains in which the relative expression level of Pletl gene is 10 times or more higher than those of the Yc#1 strain and Yc#2 strain (Y'#2, Y'#5 to Y'#8, X'#10 to Y'#13, Y'#15, and Y'#16), and there existed 5 strains in which the relative expression level of Pletl gene is 20 times or more higher than those of the Yc#1 strain and Yc#2 strain (Y'#5, Y'#6, Y'#8, X'#10, and Y'#13). The selected strains were further scaled up to a 125-mL volume Erlenmeyer flask, and thereafter dispensed and cryopreserved.

It is considered that if the relative expression level of Pletl gene of a host cell itself can be increased as in the case of the host cells obtained in the Example, production cells in which a recombinant protein is not easily reduced can be easily obtained.

[Example 7]

Confirmation that Production Strain Using Host Cell Highly Expressing Pletl can Produce Antibody that is not Reduced

It was thought that a cell producing a protein that is not easily reduced can be prepared by using a host cell in which the gene expression level of Pletl is increased, and therefore, the following test was performed.

With respect to the host cells in which the expression level of Pletl is high (X'#5, X'#6, X'#13, and Y'#10) obtained in Example 6, antibody-producing strains (BX'#1, BX'#2, BX'#3, and BY'#1) into which the antibody B was introduced were obtained according to the method shown in Example 3.

Further, in order to confirm the difficulty in reduction of these antibody-producing strains, also with respect to host cells (Xc#2 and Yc#1) in which the expression level of Pletl is low as controls, the same procedure was performed, whereby antibody B-producing strains BXc#1 and BYc#1 were obtained.

The above-prepared strains were cultured for 13 days in a $CO_2$ incubator using a 125-mL volume Erlenmeyer flask. During the culture period, a feed medium was appropriately added. The extraction of RNA was performed using mRNA catcher (Thermo Fisher Scientific, Inc.) for 50 $\mu$L of the culture broth on day 6 for each culture broth.

With respect to the obtained mRNA, the gene expression level of Pletl was measured using the RT-qPCR method in the same manner as in Example 2. With respect to three strains of each of the BX' strain and the BY' strain, the relative expression level (Fold change) was calculated using the BXc#1 and BYc#1 showing the lowest Pletl gene expression level as control cells. Further, on day 13 of culture, the susceptibility to reduction of the antibody was evaluated in the same manner as in Example 1.

The results are shown in Table 8 and Table 9.

[Table 8]

| Host | Antibody-producing strain | Relative expression level | Whether antibody was reduced or not |
|---|---|---|---|
| X'#5 (High expression of Pletl) | BX'#1 | 7 | not reduced |
| X'#6 (High expression of Pletl) | BX'#2 | 8 | not reduced |
| X'#13 (High expression of Pletl) | BX'#3 | 7 | not reduced |
| Xc#2 (Low expression of Pletl) | BXc#1 | 1 | reduced |

[Table 9]

| Host | Antibody-producing strain | Relative expression level | Whether antibody was reduced or not |
|---|---|---|---|
| Y'#10 (High expression of Pletl) | BY'#1 | 25 | not reduced |
| Yc#1 (Low expression of Plet1) | BYc#1 | 1 | reduced |

As shown in Table 8, in the three strains (BX'#1, BX'#2, and BX'#3) prepared from the host cell X' strain in which the relative expression level of Pletl is high, reduction was not observed in all the antibody-producing strains. On the other hand, in one strain (BXc#1) prepared from the Xc strain in which the relative expression level of Pletl is low, reduction of the antibody was confirmed.

Similarly, as shown in Table 9, also in the BY'#1 strain prepared from the host cell BY'#10 strain in which the relative expression level of Pletl is high, reduction of the antibody was not observed. On the other hand, in one strain (BYc#1) prepared from the Yc strain in which the relative expression level of Pletl is low, reduction of the antibody was confirmed.

From the above results, it was indicated that by using a host cell in which the relative expression level of Pletl gene is increased, a cell producing an antibody that is less susceptible to reduction can be obtained with high probability.

While the present invention has been described in detail and with reference to specific embodiments thereof, it will be apparent to one skill in the art that various changes and modifications can be made therein without departing from the spirit and scope thereof. This application is based on Japanese Patent Application (Japanese Patent Application No. 2016-256279) filed on December 28, 2016, the entire contents of which are incorporated hereinto by reference.

## Claims

1. A method for selecting a cell, comprising the following first step and second step:

   a first step: measuring the expression level of a gene in a cell, the gene being at least one gene selected from genes comprising any one of the base sequences represented by SEQ ID NOS: 1 to 16 or orthologous genes thereof; and
   a second step: comparing the expression level of the gene measured in the first step with a control value of the expression level of the gene in a control cell, and evaluating the expression level capable of suppressing reduction of a recombinant protein based on a difference therebetween.

2. A method for selecting a cell, comprising the following first step and second step:

   a first step: measuring the expression level of a protein encoded by at least one gene selected from genes comprising any one of the base sequences represented by SEQ ID NOS: 1 to 16 or orthologous genes thereof; and
   a second step: comparing the expression level of the protein encoded by the gene measured in the first step with a control value of the expression level of the protein encoded by the gene in a control cell, and evaluating the expression level capable of suppressing reduction of a recombinant protein based on a difference therebetween.

3. A method for selecting a cell, comprising the following first step and second step:

a first step: measuring the expression level of a gene in a cell, the gene being at least one gene selected from genes comprising any one of the base sequences represented by SEQ ID NOS: 1 to 16 or orthologous genes thereof; and
a second step: calculating and comparing a relative expression level and selecting a cell according to the following procedures (a) to (d);

(a) calculating a relative quantitative value by dividing the expression level of the gene in a test cell measured in the first step by the expression level of a standard gene in the test cell

a relative quantitative value = (the expression level of the gene in a test cell measured in the first step) / (the expression level of a standard gene),

(b) calculating a control value by dividing the expression level of the gene in a control cell by the expression level of the standard gene in the control cell

a control value = (the expression level of the gene in a control cell) / (the expression level of the standard gene),

(c) calculating a relative expression level by dividing the relative quantitative value for the test cell calculated in (a) by the control value calculated in (b)

a relative expression level = [the relative quantitative value for the test cell calculated in (a)] / [the control value calculated in (b)],

and
(d) comparing the relative quantitative values calculated in (a) or the relative expression level calculated in (c) among the respective test cells, and thereby selecting a cell in which each value is high or low.

4. The method according to claim 3, wherein in the second step (d), the cell in which the relative quantitative value or the relative expression level is high is a cell in which the relative quantitative value or the relative expression level is twice or more the control value, or the cell in which the relative quantitative value or the relative expression level is low is a cell in which the relative quantitative value or the relative expression level is 1/2 or less of the control value.

5. The method according to any one of claims 1 to 4, comprising knocking down or knocking out at least one gene selected from genes comprising any one of the base sequences represented by SEQ ID NOS: 10 to 16 or orthologous genes thereof in a cell before the first step.

6. The method according to any one of claims 1 to 5, comprising introducing a gene encoding a recombinant protein into the cell, thereby transforming the cell before the first step.

7. A method for obtaining a cell producing a recombinant protein, comprising selecting a cell by the method according to any one of claims 1 to 6.

8. A method for producing a recombinant protein using a cell selected or obtained by the method according to any one of claims 1 to 7 or a cell.

9. The method according to any one of claims 1 to 8, wherein the cell is a cell derived from a mammalian cell.

10. The method according to any one of claims 1 to 9, wherein the gene is Pletl gene.

11. The method according to any one of claims 1 to 10, wherein the recombinant protein is an antibody.

12. A cell capable of suppressing reduction of a recombinant protein, wherein at least one gene selected from genes comprising any one of the base sequences represented by SEQ ID NOS: 10 to 16 or orthologous genes thereof is knocked down or knocked out.

**13.** A method for increasing probability of obtaining a cell capable of suppressing reduction of a recombinant protein by using at least one gene selected from genes comprising any one of the base sequences represented by SEQ ID NOS: 1 to 16 or orthologous genes thereof as an index.

Fig. 1

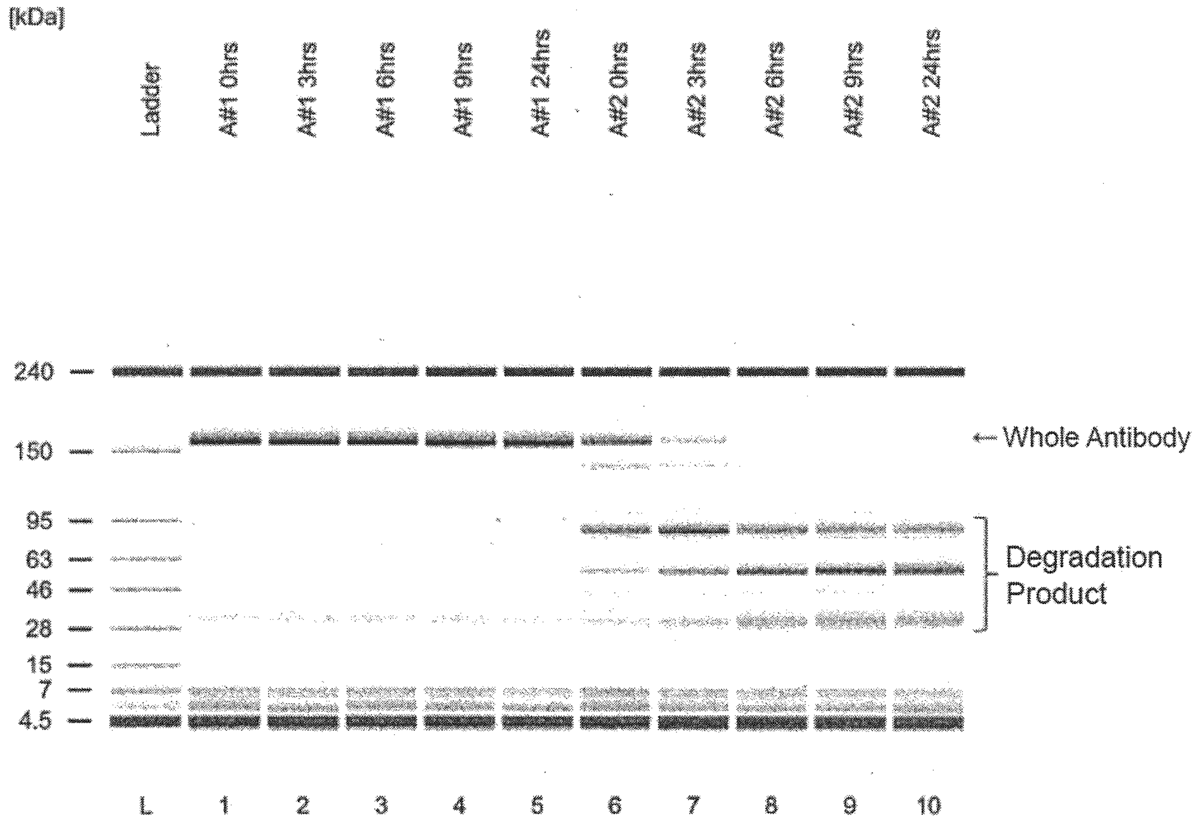
[kDa]
Ladder
A#1 0hrs
A#1 3hrs
A#1 6hrs
A#1 9hrs
A#1 24hrs
A#2 0hrs
A#2 3hrs
A#2 6hrs
A#2 9hrs
A#2 24hrs
240
150
95
63
46
28
15
7
4.5
← Whole Antibody
Degradation Product
L
1
2
3
4
5
6
7
8
9
10

Fig. 2

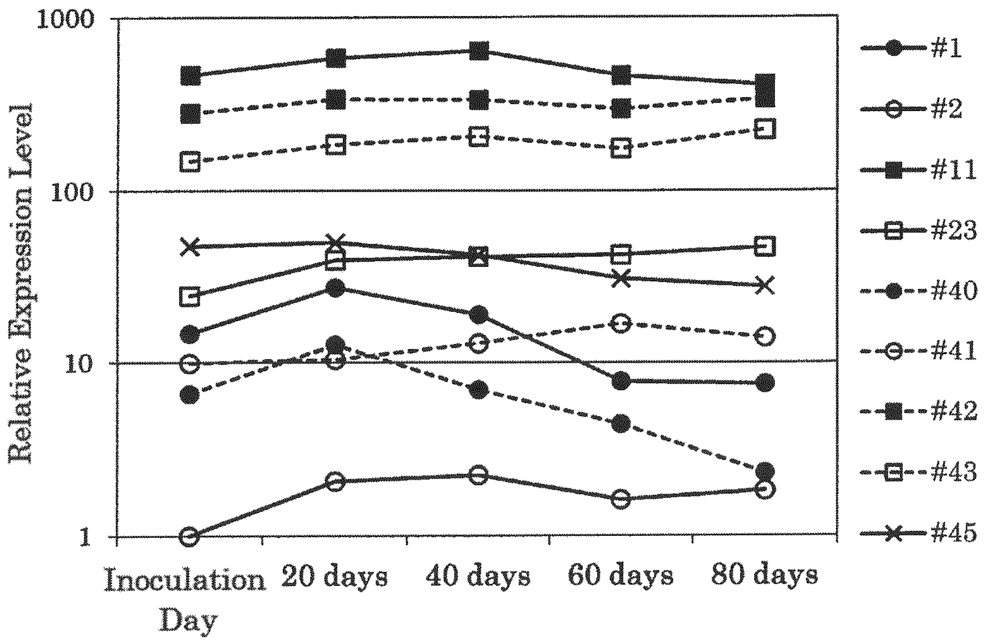
1000
100
10
1
Relative Expression Level
Inoculation Day
20 days
40 days
60 days
80 days
#1
#2
#11
#23
#40
#41
#42
#43
#45

## INTERNATIONAL SEARCH REPORT

International application No.
PCT/JP2017/046939

A. CLASSIFICATION OF SUBJECT MATTER
Int.Cl. C12N15/09(2006.01)i, C12N1/15(2006.01)i, C12N1/19(2006.01)i, C12N1/21(2006.01)i, C12N5/10(2006.01)i, C12P21/00(2006.01)i, C12P21/08(2006.01)i, C12Q1/04(2006.01)i, C12Q1/68(2018.01)i
According to International Patent Classification (IPC) or to both national classification and IPC

B. FIELDS SEARCHED
Minimum documentation searched (classification system followed by classification symbols)
Int.Cl. C12N15/09, C12N1/15, C12N1/19, C12N1/21, C12N5/10, C12P21/00, C12P21/08, C12Q1/04, C12Q1/68

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580(JDreamIII), CAplus/MEDLINE/EMBASE/BIOSIS(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br>A | TAFT, S. C. et al., Toxicity of anthrax toxin is influenced by receptor expression, Clinical and Vaccine Immunology, 2008, vol. 15, no. 9, pp. 1330-1336, p. 1330, Materials and methods | 12<br>1-11, 13 |
| X<br>A | BioProject: PRJNA72741, PREDICTED: gastrokine-1 [Cricetulus griseus], NCBI, LOCUS XP_003503622, 27 May 2016, retrieved on 28 March 2018, https://www.ncbi.nlm.nih.gov/protein/XP_003503622.1 | 12<br>1-11, 13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29.03.2018 | 10.04.2018 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2017/046939

C (Continuation). DOCUMENTS CONSIDERED TO BE RELEVANT

| Category* | Citation of document, with indication, where appropriate, of the relevant passages | Relevant to claim No. |
|---|---|---|
| X<br>A | XU, X. et al., The genomic sequence of the Chinese hamster ovary (CHO)-K1 cell line, Nature Biotechnology, 2011, vol. 29, no. 8, pp. 735-741, online methods, whole document | 12<br>1-11, 13 |
| A | KAO, Y. H. et al., Mechanism of antibody reduction in cell culture production processes, Biotechnology and Bioengineering, 2010, vol. 107, no. 4, pp. 622-632, whole document | 1-13 |
| A | TREXLER-SCHMIDT, M. et al., Identification and prevention of antibody disulfide bond reduction during cell culture manufacturing, Biotechnology and Bioengineering, 2010, vol. 106, no. 3, pp. 452-461, p. 456, right column, l. 12 to p. 457, left column, l. 10, p. 458, left column, ll. 13-27 | 1-13 |
| A | WO 2014/054744 A1 (KYOWA HAKKO KIRIN CO., LTD.) 10 April 2014, whole document, claims & US 2015/0275259 A1 & EP 2905342 A1, whole document, claims | 1-13 |
| A | US 2006/0030022 A1 (JONATHAN, B.) 09 February 2006, whole document, examples & US 6872563 B1 | 1-13 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2014129358 A
- US 6946292 B, CHO
- JP 2016256279 A

### Non-patent literature cited in the description


- *Biotechnology and Bioengineering,* 2010, vol. 7 (4), 622-632
- *Biotechnology and Bioengineering,* 2010, vol. 106 (3), 452-461
- *Bioengineering and Biotechnology,* 2015, vol. 112 (4), 734-742
- *Journal of Biotechnology,* 2012, vol. 157, 261-267
- *Genome Biol.,* 2002, vol. 3 (7), 1-11
- **YI QU et al.** *BMC Bioinformatics,* 2010, vol. 211 (11

SEQUENCE LISTING

<110> Kyowa Hakko Kirin Co., Ltd.

<120> METHOD FOR SELECTING CELL

<130> W524058

<150> JP2016-256279
<151> 2016-12-28

<160> 30

<170> PatentIn version 3.5

<210> 1
<211> 624
<212> DNA
<213> Hamster

<400> 1
atggctgtgc tccgttcctt gctgccacag ctggggctgt ttctgtgcct ggctctgtgc 60

ttttctcctg ccttgtctgc aagttataat gacccctgca tggtctttga tacaatctcc 120

accagcgaca acttgggagt cagcattacg gtggccggga gctctggtga gaacataacc 180

tacacagtgt tggttcacgt gaacagttcc gtcagtgccg tgattctgaa agcagtgaac 240

cagaacaagc ccgtgggttc ctgggttgga gcaactcagg aatgcaatga cagcagtgtc 300

ctatatcgcg tgacaccctc agacagttct ggcttccagg caacgtggat agttcctaat 360

tctgaggata tgactaaagt caacctgcat gtcttcttag ctaccggcaa tggaacagct 420

gcaatgacat ctgtgaacct gggagagcaa aaaacacctg taaccgttaa ccccacccct 480

gagatttctg agaccaacca aaccacagcc atgactacag acaggaccac agccaaaagc 540

ctggctgtca atgctctcgg cagccccctc gcaggtgcct tccacatcct gcttgttttt 600

ctcatcagca aactcctctt ctag 624

<210> 2
<211> 228
<212> DNA
<213> Hamster

<400> 2
atgataaata ctagcagggc ctcactcctg gtcttaaaac tttgccacat tctgtcttac 60

ataatacaga atcaacccag acacgacctg atcagtcagt ccagttcaaa gaaacttaca 120

tctgtaagag tgaagaaatg tccattccgt gagaatttgc aaggcaccat tagttggtgg 180

tcaatgaaat ttttaaaaac acctggtctc atcaagtgtt caatataa 228

<210> 3
<211> 162
<212> DNA
<213> Hamster

<400> 3
atgtggtcag agaagacaga gcatcctcct tacacacaga gcgtagtgct ttggggaaaa 60

aagtcaactt tacttaccag agaaaccatt accattgatg aaataaagtc atttcatcgt 120

ttactgcaca agattcattt gaagggtatg tatggatatt aa 162

<210> 4
<211> 1872
<212> DNA
<213> Hamster

<400> 4
atgagaggtc ttttctggcc actgtcactg ttgctgctct ccctccagcc ctgggaaatc 60

cagctccagt ctgcaggtcc taggtgctat actgggcccc tggatttggt gttcgtgatt 120

gatagctccc gcagcgtgcg ccccttcgag ttcgagacca tgcggcagtt tctagtgggc 180

ctcctccgta gcctggatgt ggggctgaac gccacgcgtg ttggagtgat ccagtattct 240

agccaagtgc agagcgtgtt tcccctgggc gccttctccc gtcgcgagga catggagcgc 300

gccatcagcg ccgtagtgcc tctggcgcag ggcaccatga ccgggctggc gatccagtat 360

gccatgaacg tggccttcag tgaggccgaa ggcgcccgcc caccggagga gcgagtgccg 420

cgggtcttgg tcatcgtgac cgacgggaga cctcaagacc gagtggccga agtggccgcg 480

caggcgcgcg cccgcggcat cgagatctac gcggtggggg tgcagcgagc cgatgtgggc 540

tctctacgag ccatggcttc gccgccgctg gatcagcacg tcttcttggt ggagtccttc 600

gatctcatcc aggagtttgg tctgcagttt cagggtcggc tgtgcgggaa ggacctgtgt 660

gctgagtggg gacatggctg ccaacaccag tgtgtcaatg ctccaggaac cttctactgc 720

acctgcaact ctggctacaa gctagcccca gataataaga actgtttggc catggacctc 780

tgtgctgaag gaacccacgg ttgtgaacac ctctgtgtca attccctgga ctcctatttc 840

tgtcgttgtc gagctggctt tgcactccag caggaccaga agagctgcag ggccattgac 900

tactgtagct ttggaaacca tagctgccag catgagtgtg tgaacaccct agaggggccc 960

cagtgtatct gcagagaggg tcatgacctg ctgcctgatg ggaggagctg tcgggtcagg 1020

gacttctgca atggtgtgga tcatggctgc gagttccagt gtgtgagtga gggtctttcc 1080

ttccgctgcc tgtgccctga ggggaggcga cttcaggctg atggcaagag ctgtgaccgg 1140

tgccgagagg gccacgtgga tcttgttctt ctggtggatg gctccaagag tgtgcgtcca 1200

cagaacttcg agctggtgaa acgcttcgtg aaccagatcg tggacttcct ggacgtgtcc 1260

cctgagggca cacgcatcgg gctggtgcag ttctccagcc gggtgcgcac cgagttccca 1320

ctgggccgct atggcactgc agctgaggtg aagcaggcag tcctggctgt ggagtacatg 1380

gagcgcggca ccatgacggg actggcgctg cgccacatgg tggagcacag cttctcagag 1440

gcgcagggtg cgcggcctcg tgacctcaac gtgcctcgtg tgggcttggt gttcactgat 1500

ggccgctctc aggatgacat ttcagtgtgg gcagctcgtg ccaaagagga aggcatcgtc 1560

atgtatgctg tgggcgtggg caaggccgtg gaggaagagc tgcgtgagat cgcatccgaa 1620

ccttcggagc tgcatgtgtc ctacgctccg gacttcagca ccatgactca tctgctggag 1680

aacctcaaag gcagcatttg cccagaggag ggcatcagcg ctgggacaga gcttcggagt 1740

ccctgcgaat gcgaaagcct cgtggaattc cagggccgca cgctgggggc gctcgagagc 1800

ctgacacgga acctggccca gctgaccgag cgcctggagg aactggagaa ccagctgact 1860

agccggaagt ga 1872

<210> 5
<211> 153
<212> DNA
<213> Hamster

<400> 5
atgctgttct caagtgcacc tgtaactcct aatgttttct ttacctgcga atggagtgaa 60

caaggtagga caaaccccag acccgcagga catgctggga cacctgggag gccaaggtct 120

gcagttggaa acctgagtat ctctgagact tga 153

<210> 6
<211> 264
<212> DNA
<213> Hamster

<400> 6
atgacgttct ccaccaagaa ggtccgactc tgccggttcc gccgaacatg ctccttagct 60

agcggccaac agaaagaaaa aggaacaagc gtaagaaaac ttggagagga aaaagcgacc 120

attgtctgca gctgctggag acaacagcac ggtgggtgca gggacggaat gtttgcggac 180

acttggccaa taggattaat aaacacgacc tcaaaaacca gaaagccagt cagtccctcg 240

tccttggccc aggcccagct gtaa 264

<210> 7
<211> 2712
<212> DNA
<213> Hamster

<400> 7
atgagggatc tgccaacact gccttgccac tgggtttgtc tactctggtc cttctgcagc 60

tttcaggtgt gtggcaccca gcccagagcc caggaacatc cagggtttgc agtcttggct 120

tctgcttctc attactggcc attggaaaat gtggacggga tccttgagct tcaggacaca 180

actggagcgt tgcgaaccca caacctcact gtgcctccct cccacaatgc tacctttgtc 240

tttaccaatg attctgccta ctccaacttc tctgcgactg tagatattat ggaagggaaa 300

gtcaacaaag gtatttacct caaggaggag aagggtgtga cattcctcta ctatggcagc 360
tacaagtcat cctgtatcag caacccggcc cagtgtggac ctgagggagt gacattttca 420
ttcttctgga agactcaaga ggagcagcct cgaccagccc cctacgctta tgggggacag 480
gtcatctctg atggtttcaa agtctgctct agtggtggaa agggctctgt ggagctgtac 540
acccgggata attccatgac atggaaggcc accttcaacc ctcctggtcc ttactggact 600
catgtcctgt tcacgtggaa atccaaggag ggcctaaaag tctatgttaa tgggactctg 660
agcacctcag acccgagcgg gaaagtgtct cacacctatg gtgagcccca tgtcaacctg 720
gtcatagggt ctgagcagga ccagaccaag cgctacgaga acggagcttt cgatgagttc 780
atcatctggg aacgtgcact cactcccgat gagatcaaga tgtacttcac agctgctatt 840
ggtaaacact ctctgttgtc ttcaacacca ccagcgatgc ctacagcaca ctccatggtg 900
cccacggatg cctaccaccc aatcatcacc aacctgacag aggagaggaa atgcttccag 960
agacctgaaa ccgtgcttcg ataccttcaa aatgtgtccc tcagcttgcc caataaatcc 1020
ctctcagagg aaacagcgct aaacctcaca cagaccttct taagaactgt gggtgaggtg 1080
cttctactgc ccagctggac ccatgtatca gaggacaaca ccatgacgct gggcctggtt 1140
gacaccatcg ataccgtcat gggtcacata tcagccaacc tgcagtccag agaaccccat 1200
atcaccctca caggctcttc ctccatggca gatttcttcg tggccaaggt ccttccccca 1260
gcactaagtg ccccccacta tagattccca gcccatggtc acagctacat tgagattcca 1320
agagaggccc ttcacggcaa agcctggacc acgattgtcg gccttctcta ccactccatg 1380
cactattacc tgaacaacat ccccccagcc agcacaaaga ttcctgaggc tgtgaactgc 1440
agagactgcc tgctgtctgt cgccagccac ctgatatccc tggaggtgtc ccccaccgcc 1500
actctgtccc agaacctatc gggctctcct ctgatcactg tccatctcag gcacaaactg 1560
actcataagc agtatgtcga tgccaccaac gagagcaacg acctcttcct gtattgtgcc 1620
ttccttaact tcagctccgg ggaaggcatc tggtcaagcc agggctgtgc gctcagagag 1680
ggaaacctca cccactcagt gtgccactgc acacatctca ccaactttgc catcctcatg 1740
caagtggtcc cactacagct cacccgtgga caccaggtgg cgctgtcgtc catcagttat 1800
gttggctgct ctctgtcagt tctctgcctg gctgccacgc tggtcacctt cgcagtactg 1860
tcatctgtca gcaccattcg gaaccagcgt taccacatcc atgccaatct atccttcgct 1920
gtcctggtgg cccaggttct gctgctcatc agcttccgtg tggagcctgg cacagtcccg 1980
tgccaggtgc tggccatcct cctgcactac ttcttcctga gtgcctttgc ttggatgctg 2040
gtggagggac tacatctcta cagcatggtg ataaaggtct tcgggtcaga ggacagcaag 2100
catctctact actatgggat aggctggggg tgccctctcc tcatctgcat catctccata 2160
tcatcctcta tggacagtta tgggacaaat gacagttgct ggctgtcact ggggagtggt 2220

gccatttggg cctttgtggg ccctgccctg ctggttattg tggtcaacat tgttattctg 2280

gtagctgtga caagggtcat ttctcatgtc agcaccgaca actacaagat acatggggac 2340

cccagtgcct tcaagttgac agccaaggct gttgctgtgt tgctgcccat cctgggaacc 2400

tcatgggtgt ttggggtact tgccgtcagt gaccgggccc tggtcttcca atacatgttt 2460

gctgtactca actccttaca gggcctcttc atcttcctct ttcactgtct cttaaactca 2520

gaggtgagag cggccttcaa gcataagacc aaggtctggt ccctcacgag cagctcagcc 2580

cgtaccacaa acaccaaacc cttcagttcc gatatcgtaa atggcacccg gccaggcaca 2640

gcatctacaa agttaagccc atgggacaag agcagccact ctgcccaccg tgtggaccta 2700

tcggctgtat ga 2712

<210> 8
<211> 6060
<212> DNA
<213> Hamster

<400> 8
atgggggcca tgacctggtt attgccaggc atctttctag ctttgcttgc cctcaattcc 60

gaaggtgcag tcctcaagaa agtcatccgg cacaagcgac agagtggact gaacatgacc 120

cttccagagg agaatcagcc agtggtgttc aaccatgtct acaacatcaa gctgcctgtg 180

ggctctcagt gctcagtgga tctggagtca gtgaatgggg agaaagacct gaccccgacg 240

ccagagtcaa gtggaacctt ccaggaacat acagtggatg gggaaaacca gattgtgttc 300

acacaccgca tcaacatccc ccgtagggcc tgtggctgtg ctgcagctcc tgatgtgaag 360

gagctcctga gcaggctgga ggagctggag atgctggtgt cttctctacg ggagcagtgc 420

accatgggta caggctgttg tctccaacct gcagaaggcc gtctggacac taggcccttc 480

tgcagtggcc gaggcaactt cagtgccgaa ggatgtggct gtgtttgtga gccaggctgg 540

aaaggcccca actgctctga gcctgaatgt cctggaaact gcaacctaca gggccagtgc 600

cttgatggac agtgtgtctg tgacgagggt ttcactggtg aggactgcag tcagctagcc 660

tgtcccaatg actgcaatga ccagggcaga tgtgtgaatg gagtctgtgt gtgctttgaa 720

ggctatgcag ggttgactg tggcctggaa gtctgccccg tgccctgcag tgaggagcac 780

gggacctgtt tggatggcag gtgtgtgtgc aaagatggct ttgctggtga tgattgcaat 840

gagcccctgt gcctcaacaa ctgctacaac cgtgggcgat gtgtagagaa cgagtgtgtg 900

tgtgatgagg gcttcacagg cgaggactgc agtgagctca tctgccccaa tgactgcttc 960

gaccgcggtc gctgtgtcaa tggcacctgc tactgtgagg aaggttttac aggtgaagac 1020

tgtggtgagc tcacctgccc caataactgc cagggccatg gccagtgtga ggagggacag 1080

tgtgtttgtg atgagggctt tgccggtgca gactgcagtg aaaagcggtg tcctgaagat 1140

tgtcaccacc gtggccgctg cctcaatggg caatgtgagt gtgatgatgg gttcatgggg 1200
gctgactgtg gggatctaca gtgtcccaac ggctgcagtg ggcatggccg ctgtgtcaat 1260
ggacaatgtg tgtgtgatga gggctacact ggagaagact gcagtcagca gcgatgtccc 1320
aatgactgcc acaaccgagg cctgtgcgta cagggcaagt gcatatgtga acaaggcttc 1380
aagggctttg actgtagtga gatgagctgt cccaatgact gccatggcca tggccgctgt 1440
gtaaatggca tgtgcatctg tgatgatgaa tacactgggg aagactgcag agaccatcgc 1500
tgtccccggg actgcagcca gcggggacgc tgtctggatg gacaatgtat atgtgaggat 1560
ggctttactg gccctgactg tgctgagctc tcctgcccca atgactgtca tggccatggc 1620
cgctgtgtga atggccagtg catctgccat gagggcttca ctggcaaaga ctgcaaagag 1680
caaaggtgcc ccagtgactg ccatggccaa ggccgctgtg aggatggcca gtgcatctgt 1740
catgaaggct tcacaggcct ggactgtggg cagcgctcct gccccaatga ctgcagcaac 1800
caagggcaat gtgtttcagg ccgctgcatc tgcaatgaag gttacagagg ggaagactgt 1860
tctgaagtgt ctcctcccaa agaccttatt gtaacagaag taacagaaga gaccgtaaac 1920
ctggcctggg acaatgagat gcgggtcacc gagtacctca ttatgtacac acccacccat 1980
gctgatggcc tagagatgca gttccgtgtg cctggggacc agacatccac cactattcga 2040
gagctagagc caggggtgga atacttcatc cgtgtgttcg ccatcttgga gaacaagagg 2100
agcatccctg tcagtgccag agttgctacc tacttgcctg cacctgaagg actaaaattc 2160
aagtctatca aggagacatc tgtggaagta gagtgggacc ctctagacat tgctttcgaa 2220
acctgggaga tcatcttccg gaatatgaat aaagaagatg agggagaaat caccaaaagc 2280
ttgaggagac cagagacctc ttaccgacaa actggccttg ctcctgggca agaatatgaa 2340
atatctctgc acattgtgaa aaacaacacc agaggccctg gcttgaagaa agtgaccaca 2400
acccgcttgg atgcccccag ccagattgag gtgagagatg tcacagacac cacagcactg 2460
atcacctggt tcaagcccct ggctgagatt gatggcatcg agctctccta tggcatcaag 2520
gacgtgcctg ggaccgcac caccatcgat ctcacacatg aagagaacca gtactccatt 2580
gggaacctga agcctgacac tgagtatgag gtgtcccttg tctcccgaag ggtggacatg 2640
gcaagcaacc ctgccaagga gaccttcacc acaggcctgg atgcgcccag gaatcttcgc 2700
cgtgtctccc agacagataa cagcatcacc ctggaatgga ggaatgtcaa agctgacatt 2760
gacagttaca gaattaagta tgcacctatc tcgggaggtg accatgctga ggttgatgtt 2820
ccaaagagcc aacaagctac aaccaaaacc acactcacag gtctgaggcc aggaactgaa 2880
tatggcgttg gtgtttctgc tgtgaaggga gacaaggaga gtgacccagc aaccatcaat 2940
gctgccacag aaattgatgc acccagggac tttcaggtat ctgaaaccac acaggacagt 3000

ctgactctac tctggaaaac accactggcc aagtttgatc gataccgcct caactacagc 3060
ctccccacag gccagtcaat agaggtccag ttgccaaagg atgccacctc ccatgtcctg 3120
acagatctgg agccagggaa agaatatact gtcctcctca ctgccgagaa gggcaggcac 3180
aagagcaagc ctgcacgtgt gaaggcatcc acggaacaag ctccttccct ggaaaatctc 3240
accgtgactg aggcaggctg ggatggcctc agactcaact ggactgcgga tgacttggcc 3300
tatgagtact ttgtcattca ggtgcaggaa gccaacaagg tggaggctgc tcacaacttc 3360
acagtgctcg gcaacctccg ggctacagac atcccgggtc tcaaggctgc cactccctat 3420
agagtctcca tctatggggt agcccggggc tctagaacac cagtgctctc tgctgaggct 3480
tccacaggga aaactcccaa tttgggagag gtctctgtcg ctgaggtggg ctgggatgcc 3540
ctcaaactca actggactgc tccagaaggg gtctatcaga actttttcat tcaggtgcta 3600
gaggctgaca cgacccagac cgtccagaac ctcacagtcc ctggaggact gaggtcagtg 3660
gacctgcctg ggctcagagc agccactcac tatcacatca ccatccgagg ggtcactcag 3720
gacttcagca cagcccctct ctctgttgaa gtcttaacag aggagctccc tcatctggga 3780
ggcttatcag tgactgaggt cagctgggat ggcctcatac tcaactggac cacagacgat 3840
ctggcctatg agcactttgt tatccaggtg caggaggcca acaatgtgga ggctgctcag 3900
aacctcacag tgcccggtag catcagggct atggaaatcc caggcctcaa ggctgccact 3960
ccttatagag tctccatcta tggggtgatc cagggctata gaacaccaat gctctctgct 4020
gacgcctcca cagccaaaga acctgaaatt ggaaacttga atgtttctga cgtaactcct 4080
gagagcttca atctctcctg gacagcgact gatgggatct tcgacatgtt tactattgaa 4140
attattgatt ccaataggtt gctgcagatg gcagagcata acatatctgg tgctgaacga 4200
actgcccaca tctcagggct tcccccctagt actgatttca ttgtctacct ctctggaatt 4260
gctcccagca tccggaccaa aaccatcagt accacagcta ccacagaagc tgaaccagaa 4320
gttgacaacc ttctagtttc agatgccact ccaggtggtt tccgtctgtc ctggactgct 4380
gatgaaggga tattcgacag ttttgttctc aggattagag ataccaaaaa gcagtccgaa 4440
ccacaagaaa taatcctccc ttcccctgaa cgtaccaggg acataactgg tctcagagag 4500
gccactgagt atgaaattga actctatgga ataagcggtg gaaggcgatc ccagccagtc 4560
agtgccatag caacaacagc catgggttct ccgaaggaaa tcatgttctc agacatcact 4620
gacaatgcag ccacagtcag ctggaaggca cccactgccc aagtagagag cttccggatt 4680
acctatgtgc ctatgacagg aggtgccccc tccatggtga cagtggatgg aactgatact 4740
gagacccgtc tggtgaagct cacccctggg gtggaatacc atgtcagcgt tattgccatg 4800
aagggctttg aagaaagtga tcctgtctcg gggtcactaa ccacagctct ggatggtccg 4860
tctggcttgc tggcagcaaa catcacagac acagaagcct tggcactgtg gcaaccagcc 4920

attgccactg tggatagtta tgtcatctcc tacacagggg agagagtgcc agaagttaca 4980

cgcacagttt ctgggaatac ggtggagtat gagctacatg acctggagcc tgccacagaa 5040

tacacactga ggatctttgc agagaaagga caccagaaga gctctgctat tactaccaag 5100

ttcaccacag accttgattc ccaagagac ttgactgcta ctgaggttca gtcggaaact 5160

gccctcctca cctggcgacc tccccgggca tcaatcaccg gttacctcct ggtctatgaa 5220

tctctggatg gtacagtcaa ggaagtcatt gtgggaccgg ataccacctc ctacagcctg 5280

gcagacctga gtccatccac ccactacaca gtgaggatcc aagcattgag tgggtcccta 5340

aggagcaagt tgatccaaac catctttacc acaattggac tcctgtaccc attccccagg 5400

gattgttctc aagcaatgtt gaatggagac accacctctg gcctctacac catctatata 5460

aatggtgaca agactcaagc tctagaagtc tactgtgata tgacctctga tggaggtgga 5520

tggatcgttt tcctgagacg caaaaatgga cgtgaggact tctatagaaa ctggaaggcc 5580

tatgctgctg gttttgggga ccgcagagaa gaattctggc ttggactgga taacctgagc 5640

aaaatcacag cccaagggca gtatgagctc cgggtggacc tacaagacca tggggaatca 5700

gcctttgctg tgtatgatag gttcagtgtg ggagatgcca agagtcgcta caagctgaaa 5760

gtagaaggat acagtggaac agcaggtgac tccatgaact accacaatgg cagatccttc 5820

tccacctatg acaaggacac agactcagcc atcaccaact gtgctctgtc ctacaaagga 5880

gctttctggt ataagaactg ccatcgtgtc aacctaatgg gcagatatgg ggacaataac 5940

cacagtcagg gcgttaactg gttccattgg aagggccatg agtactcaat ccagtttgct 6000

gagatgaaac tgagacccag caacttccga aatcttgaag gcaggcggaa gcgagcataa 6060

<210> 9
<211> 312
<212> DNA
<213> Hamster

<400> 9
atgaccaaca gccagatgag gtggtcatat ccaaatcagg ctttagctga ccagctacca 60

aacaaaagtt cgcagcatca gtttgcttcc tttcatggga gaacagaatt agaagccaag 120

atcccggtgc tacacctgtt tattgctgct ggagtgacat tcctttcaga tcctccaagc 180

ttcaatgcaa agcatcacat cagcccaggc gtaagccctc atctgagaaa tcccaccacc 240

gtctgcaccc atgttaaggg gacgggtcct agtgtctcca gctctaaccc attgccaagt 300

ggaccgtgct aa 312

<210> 10
<211> 4401
<212> DNA
<213> Hamster

<400> 10

atgatgagct ttgtgcaaag tgggacttgg cttcttctcg ccctgcttca tcctactttc 60

atttgggcac agcagtccag tgtagacgaa tcgggatgca gccaccttgg tcagtcctat 120

gagtccaggg atgtttggaa gccagaacca tgtcaaatat gtgtctgtga ctccggatct 180

gtcctctgcg atgacataat atgtgatgag gatccactag actgccccaa cccagagatc 240

ccatttggag aatgttgtgc aatatgccca cagccttcaa cagctgctcc tgtacctcct 300

gatggtcacg gacctcaagg ccccaaagga gatccagggc ctcctggcat tcctgggaga 360

aatggtgatc ctggccttcc agggcaacca ggtctccctg gacctcctgg ctctcctgga 420

atctgtgaat catgtccaac tggtggtcag aattattctc cccagtatga ctcatacgat 480

gtcaagtctg gagtaggagc aggaggactt ggtggtggct atcctggtcc agctggtccc 540

ccaggccctc ctggtccccc tggctcagtt ggacatcctg gctcccctgg ttctcctgga 600

taccaaggtc cccctggtga acctggtcaa gctggtcctt caggcccccc aggacctcct 660

ggtgctattg gtccatctgg tcctgctgga aaggatggag agtcaggaag accgggacga 720

cctggagaac gtggactgcc tggtcctcca ggtatcaaag gcccaagtgg catgcctgga 780

ttccctggta tgaaaggaca cagaggtttt gatggacgaa acggagaaaa gggtgaaacc 840

ggtgctcctg gattgaaggg tgaaaacggt ctcccaggtg acaatggagc tcctggcccc 900

atgggtccca gaggggctcc tggtgagaga ggacggccag gccttcctgg agctgcaggt 960

gctcgaggca atgatggtgc tcggggcagt gatggccaac caggtccccc tggtcctcct 1020

ggaaccgcag gattccctgg atcccctggt gctaagggtg aagttgggcc tgcagggtcc 1080

cctggctcaa atggctctcc aggacaaaga ggggaacctg gaccacaggg acacgctggt 1140

gctcaggggc ctcctggccc tcccgggaat aatggcagtc ctggtggcaa aggtgaaatg 1200

ggtcctgctg gcattcctgg agctcctgga ctaatgggag ctaggggtcc ccctggacca 1260

gctggcacta atggtgcacc cgggcaacga ggtccttcag gtgaacccgg caagaatggt 1320

gccaaaggag agccaggagc tcgtggtgaa cgggggggaag ctggttcccc aggaatccca 1380

gggcctaagg gtgaagatgg caaagatgga tcacctggag aacctggtgc aaatggactt 1440

ccaggaactg ctggagaaag gggtgctcct ggcttccgag gacctgcagg gccaaatggc 1500

atcccaggag aaaagggtcc tgctggggag cgtggtgccc caggtcctgc agggccccga 1560

ggagtggctg gagaacctgg ccgagatgga aacccaggag gcccaggaat gaggggtgtg 1620

cccggaagcc caggaggacc aggcaatgat gggaaaccag gacctcccgg aagtcaagga 1680

gaaagtgggc gccctggtcc tcctggtcca tctggccccc ggggtcagcc tggtgtcatg 1740

ggtttccctg gccctaaagg aaatgatggt gctcctggca agaatggaga acggggtggc 1800

cctggaggac ctggccttcc aggtcctgct ggaaagaatg gtgaaactgg acctcagggt 1860

cccccaggtc ctactggccc atctggtgac aagggagaat ctggaccccc tggtccacaa 1920

ggattacaag gaatacctgg taccagtggt cctccaggag aaaatggaaa accaggtgaa 1980

ccagggccaa agggagaagt tggtgcacct ggagttcccg gggcaaggg tgatgccggt 2040

gcccctggag aacgtggacc acctggaact gcaggggtcc ctggtcttag aggaggagct 2100

ggacccccctg gccctgaggg aggaaagggc cctgctggcc cccctggtcc tcctggtact 2160

tctggttctc ctggtctaca agggatgcct ggagagagag gaggtcctgg gagtcctggt 2220

ccaaagggtg aaaagggtga accaggggggt gccggtgctg acggagctcc aggaaaggat 2280

gggccaaggg gtcctactgg tcctattggt cccccctggcc cagctggtca gcctggagat 2340

aagggtgaag gtggtgcccc tggacttccg ggtatagctg gacctcgagg tggccctggt 2400

gagagaggtg aacatgggcc tccaggacct gctggcttcc cgggtgctcc tggacagaat 2460

ggtgaaccag gcgctaaagg agaaagaggt gcccctggag agaaaggaga aggaggccct 2520

cctggacctg caggacttcc tggaggttct ggacctgctg gtcctcctgg tcctcaaggt 2580

gtcaagggtg aacgtggcag tcctggtggt cctggtgctg ctggctttcc tggtggacgt 2640

ggtcttcctg gtcctcccgg caacaatggt aacccaggcc ccccagggcc tagtggtgct 2700

cctggcaagg atggtcctcc aggtcctgct ggtaacagtg gttcccctgg caacccggga 2760

gtagctggac caaaaggtga tgctggtcag cctggagaga agggaccacc tggtgctcaa 2820

ggccctccgg gatctccagg cccacttgga attgcaggac ttacaggagc acgaggtctt 2880

gctggaccac caggcatgcc aggtcctagg ggtagccctg gccctcaagg tatcaagggt 2940

gaaagtggaa aaccgggagc cagtggccat aatggagaac gtggtcctcc tggaccccaa 3000

ggtcttcctg gtcaacctgg tacagctggt gaacccggaa gggatggaaa ccctggatca 3060

gatggtcagc caggacgaga tggatctcct ggtggcaagg gtgatcgtgg tgaaaatggc 3120

tctcctggtg ccccaggcgc tcctggtcat ccaggaccac ctggtcctgt tggtccagct 3180

gggaaaagtg gtgacagagg agaaacgggg cctgctggtc cttctggtgc tccaggtcct 3240

gctggatctc gcggtcctcc cggtccccaa ggtcctcgag gtgacaaagg tgaaactggt 3300

gaacgtggct ccaatggcat caaaggacat cgaggattcc ctggcaatcc aggtccccca 3360

ggttctcctg gtgctgctgg tcaccagggt gcagttggta gtcctggacc tgcaggtccc 3420

agaggaccag ttggaccaca tgggcctcct ggaaaagacg gaactagtgg gcatccaggt 3480

cccattggac caccaggacc tcgaggcaac agaggtgaaa gaggatctga aggctcacca 3540

ggccaccctg ggcagccagg cccccctgga cctcctggtg cacctggtcc ttgctgtggt 3600

ggaggtgctg ctgcccttgg aggtggtggt gaaaagtctg gtggattttc accatattat 3660

ggagatgaac caatggattt caagattaac accgaggaga ttatgtcttc actcaagtct 3720

gttaatggac aaatagaaag cctcattagt cctgatggtt ctcgtaaaaa ccctgctcgc 3780

aactgcagag acctgaaatt ctgccatcct gatctcaaga gcggagaata ttgggttgat 3840

cctaaccaag gttgcaagat ggatgctatc aaagtattct gtaacatgga aaccggggag 3900

acatgcataa atgctagtcc tatgactgtg ccacggaaga actggtggac agatgcctct 3960

gctgaaaaga aacatgtttg gtttggagaa tctatgaatg gtggatttca gttcagttat 4020

ggcaatcctg atcttcctga agatgtcctt gatgtacagc tggcttacct cagactcctc 4080

tccagccggg cttcccagaa cattacatac cactgcaaga acagcattgc ctacatggat 4140

caggccagtg gcaatgtaaa gaagtctcta aagctgatgg gatcaaatga aggggaattc 4200

aaggctgaag gaaacagcaa attcacttac acagttctgg aggatggctg tgctaaacac 4260

actggggagt ggagcaaaac agtcttcgaa taccgaacgc gcaaggccct gagactgccc 4320

atcatagata tcgcacccta tgacatcggt ggtcccgatc aagaatttgg tgtggacatt 4380

ggccctgttt gctttttata a 4401

<210> 11
<211> 666
<212> DNA
<213> Hamster

<400> 11
atggcgggaa agccagtcct tcactacttt gatggcgggg gcagaatgga gcctgtccgg 60

tggctcctgg ctgcagcggg agtagagttt gaagaaaaat ttctgaaaac tcgggatgac 120

ttggcaaggt taagaaatga tgggagtttg atgttccagc aagtgcccat ggtggagatt 180

gacgggatga agctggtgca gaccagagcc attctcaact acattgcctc caaatacaac 240

ctctatggga aggacatgaa ggagagagcc ctcattgaca tgtatgcgga aggtatagca 300

gatctggatg aaatagttct ccatcaacct tatattcccc aagaggagaa agaggcaaac 360

cttgccaaga tcaaggacaa agcaaggaac cgttacttcc ctgcctatga gaaggtgtta 420

aagggccatg gacaagatta tctcgttggc aacaggctga gcagggctga tgtttacctg 480

gttgaacttc tctaccatgt ggaagagctg gaccccagcg ttttggccaa cttccctctg 540

ctgaaggcac tgagaaccag agtcagcaac ctccccacag tgaagaagtt tcttcagcct 600

ggcagccaga ggaagcctta tgaggatgag aaatgtgtag aatcagcaat gaagattttc 660

agttag 666

<210> 12
<211> 1824
<212> DNA
<213> Hamster

<400> 12
atgtggttac ggtctctggt caaacagtta gagcgagggg aagcctctgt ggtcgatctt 60

aagaagaacc tggaatatgc agcaacagtg ctagaatctg tgtacattga tgaaacaagg 120

aggcttctgg atacagagga tgagcttagt gacattcagt cagatgctgt gccttctgag 180

gtccgagact ggctggcctc caccttcaca cgacagatgg ggatgatgct aaggagaagt 240

gatgagaagc ccaggttcaa gagcatcgtc catgcagtgc aagctgggat atttgtggag 300

agaatgtata ggcggacatc aaacatggtt gggttgagtt atccaccagc tgttattgat 360

gcgttaaagg atgtggacaa atggtccttc gatgtctttt ctctcaatga tgccagtgga 420

gatcatgcgc tgaagttcat tttctatgaa ttactcactc gctatgacct gatcagccgt 480

tttaagatcc ccatttctgc acttgtctca tttgtggagg ccctggaagt ggggtacagc 540

aagcacaaaa acccttacca taacctgatg catgccgctg acgtcaccca gactgtgcat 600

tacctcctct ataagacagg agtagcaaac tggctgacag aactggagat cttcgcaata 660

atcttctcgg ctgccatcca tgactatgaa cacaccggaa ctaccaacaa tttccacatt 720

cagacccggt cggatccagc tatcttgtac aatgacagat ctgtgctgga gaatcaccac 780

ttgagtgcag cttaccgcct tctgcaggaa gacgaggaga tgaatattct ggtcaacctc 840

tcaaaggatg actggaggga gtttcgaacc ttggtaattg agatggtaat ggccacggat 900

atgtcctgtc atttccagca aatcaaagcc atgaagacag ccctgcagca gccagaagca 960

attgagaagc caaaagcctt atctctgatg ctgcacacag cagacatcag ccatcctgca 1020

aaagcatggg acctgcacca ccgctggacc atgtctctcc tggaggagtt cttcagacag 1080

ggtgacagag aagcagagct ggggctgcca ttttctcctc tatgtgacag aaagtcaacc 1140

atggttgctc agtcacaagt gggttttatt gacttcattg tggagcccac cttcactgtg 1200

ctcacggata tgactgaaaa aattgtgagt ccattaatcg atgaaacatc ccagactggt 1260

gggacaggcc agaggcgctc aagtttgaac aacatcagtg cctcagatgc aaagcgacca 1320

ggtgtcaaga gttctgggtc agaaggaagc gctcccatca acaattctgt catccctgtt 1380

gactataaga gttttaaagc cacctggact gaggtggtgc acgtcaatcg ggagcggtgg 1440

cgggccaagg tgcccaaaga agagaaagcc aagaaggaag ctgaagagaa ggctcgcctg 1500

gctgctgagg aaaagcaaaa ggaaatggaa gcccaagacc aaactgaaca aggcaaagct 1560

gagaaaaaga catctggaga aacgaaaggc caagtcaatg gagcgcgtac aaacaagggg 1620

aagagcccca aaggtgacaa ggccggagag aaacagcaga atggtgactt gaaagagggt 1680

aaaaataagg cagataagaa ggatcactcc agcaccggaa atgattcaaa gaaaacagat 1740

ggtacaaaga agcgttctca tgactcacct gctccaagca ctagttccac aagtcgcatt 1800

accttgccag gagactatgg gtaa 1824

<210> 13

<211> 1599
<212> DNA
<213> Hamster

<400> 13
atggaccgcg cggggtgtct gggtgcgggc ctgcggggac tctgcgtggc tgcgctcgtg 60

ctcgtgtgcg ccgggcacgg ggcacgccgc gaggacgggg gaccagcttg ctatggggga 120

ttcgatctct acttcatcct ggacaagtca ggaagcgtgc tgcaccactg gaatgaaatc 180

tactacttcg tggagcagct ggctcataga ttcatcagcc cacagctgag gatgtccttc 240

atcgtcttct ctactcgagg gacaacctta atgaaactaa ctgaggacag ggaacagatc 300

cgacaaggcc tagaagaact ccagaaagtt ctgccaggag gagacactta catgcatgaa 360

ggatttgaaa gggccagtga gcagatttac tatgaaaaca gccaaggata caggacagcg 420

agtgtcatca ttgcactgac agatggggaa ctgcatgaag atctcttctt ctactcagag 480

agggaggcta acagatcccg aaaccttggc gcaattgttt actgtgttgg tgtgaaggat 540

ttcaatgaaa ctcagttggc tcggattgca gacagtaagg accatgtgtt tcctgtgaac 600

gatggcttcc aggctcttca aggcatcatc cactcaattc taaagaaatc ctgcatcgaa 660

attctagcgg ctgaaccatc taccatatgc gcaggagaat cttttcaagt tgttgtaaga 720

ggaaacggct tccgacacgc ccgcaacgtg gacagggtcc tctgcagctt caagatcaat 780

gactcagtca cactcaatga gaagcccttt gctgtggaag acacttactt gctgtgccca 840

gcacccatct tgaaagaagt tggcatgaaa gctgcactgc aggtcagcat gaatgatggc 900

ttgtctttca tctccagttc cgtcatcatc accaccacac actgttcaga tggctccatc 960

ctggcaattg ccctgctgat cctcttcctg ttgctggccc tggcactgct ctggtggttc 1020

tggcctctct gctgcacagt gatcatcaag gaggtccctc cacccccctgt tgaggagagt 1080

gaggaagaag acgatgatgg cctgccaaag aagaaatggc caacagtaga tgcctcttac 1140

tacggaggac gaggagtcgg aggcattaaa aggatggagg ttcgctgggg agagaagggc 1200

tccacagaag aaggtgctaa gttggaaaag gcaaagaatg caagagtcaa gatgccagag 1260

caagaatatg agttcccgga gcccgcaat ctcaacaaca acatgcgccg gccctcttcc 1320

ccccggaagt ggtactcgcc catcaaggga aaactcgatg ccttgtgggt tctgctaagg 1380

aaaggctatg accgagtgtc cgtgatgagg ccacagccgg gagatacggg gcgctgcatc 1440

aacttcacca gagtgaagaa cactcaggca gccaagtacc cactaagaag gttccagaga 1500

tggctacact gccaagatgc tctcaaccag attgtgtccc atggagacca ggaagaaagt 1560

catttccgag aacggaatgc agcattggat aagaaataa 1599

<210> 14
<211> 678
<212> DNA

<213> Hamster

<400> 14
atggcgttta caagtatgtc cacagagaag agtaagtgct ctgggagggt ccggcacaag 60

ctaattgtga tcggctcacc ttccacagca ctggctccgg ggttctactc aggccaagca 120

gtctctgggc tccagaaggt ggtcttcgtc ggcctgcttg ggctcctcat agcgtctggt 180

ttttcttaca ctatcaacag caacaatgaa agcaacgtag gaggaagtgg tccgcaggca 240

gtgagcatca acaaccagca caatgtggcc aacgtcgaca gcaacaatgg ctggggctcc 300

tggaatgccc tctgggatta tgaaaatagt tttgctgcaa ccagaatctt tgccaagaaa 360

tcgtgcattg tgcacaaaat gaacaagaat gtcatgccct ctcttcaaga actcgacaca 420

ctggtcaagg agcaaaagga taaagagcct gaaggagcaa ctcccaagga attgatgttc 480

tccatcaacc ctaccagagt ggaggacctg agtacattcg ggccaaagat tgctggcatg 540

tgccagggca tcccaaccta tgtagccgag gagattccag gaccgaacca gcctttgtac 600

gcacagaagt gcttcactgc taatatactg tggatcatca agttgtcctt ctgtggaacg 660

tcagaggaaa catattag 678

<210> 15
<211> 924
<212> DNA
<213> Hamster

<400> 15
atggccatgg accagagcac gcgggatgct aggcatccct cagttactgt gcgccctgcg 60

gatgctgcgc tgcctgcggt tgctgcgctc ctcgcagaaa cagtgcgtcc agcggatgtt 120

gagagcactg aggatgctgc agaccccagg gttaatgttc aagaccccga ggccgcgttg 180

ctgtccgccc cgcactcgtg tttccgctgt cagctgctct actgggtagg cgccttgatg 240

ttgctgctcg ggggttgtctg tgttccgatc gccctcttca cccgcatccc gacccggccg 300

gctctcacag tcaccacctc gcctaccccg gggaactcag agaagccttc aaacctgacg 360

gtcactcctg ttcctcgaaa tagctgcccg aaggctacag ggcaggggtc ttccgtgttc 420

gccattcttc aggctaaaga ggaagtaaag ctggagccca ataggatcct gaagtggcat 480

agccaagaag gagctgggat ctcaaaaccg ctccagggtc tgaggtacga caacgtcaca 540

aacgagttgg tggtgaacga aagtgggctc tactatgtga ttttgcaact gaagctcagg 600

cctgtgttaa aaaacacaga ccgcaaggtg cggggtcagg tctctcttgt tctgcaactg 660

aatcccccga tagagcgccc tgacaacttg gccctgactg tggacctatt cccttgctcc 720

atggagacca acttagtgga aggctcctgg agtcatctga taccccctgaa ggctgacgac 780

cgcctcagtg tgaatctgcg agcctatctg tatggagctc aggaggcata caaagactgg 840

gagctctccc agactgctat caccagcttt gtgctcttcc ttgtgccaac tgacacccca 900

caggaattgc catccatacg ataa 924

<210> 16
<211> 3081
<212> DNA
<213> Hamster

<400> 16
atgactgcta tcaagatgct tcagggttct tttcctgtgt tcctgctggg ggcgctcttg 60

ggagtcctcc atgctcagca gcaggaagtc atctcacccg acatctctac cattgacagg 120

aacaacaact gtccagagaa ggctgactgc ccagtcaatg tgtacttcgt gttagacacc 180

tcagagagcg tggccatgca gtcccccaca gacagcctgc tttatcatat gcaacagttt 240

gtgcctcagt tcatcagcca gctgcagaac gaattttacc tggaccaggt ggccctgagc 300

tggcgttacg gtggcctgca cttctccgac caggtggagg tgttcagccc accaggcagt 360

gaccgggcct ccttcactaa gagcctccaa agcatccgct ccttccgcag aggcaccttt 420

actgactgcg ccctggccaa catgacacag cagatccggc agcatgtagg ccgaggggtg 480

gtcaactttg ctgtggtcat caccgatggc cacgtcacag gcaatccgtg tgggggcatc 540

aaaatgcaag ctgagcgcgc ccgtgaggag ggtatccggc tctttgctgt ggcccctaac 600

aggaacctaa atgagcaagg cctgcgagac atcgctaaca ccccacatga gctctaccga 660

aacaactatg ccaccatgag gcctgactct actgagattg accaggatac catcaaccgc 720

attatcaagg tcatgaaaca tgaagcctat ggagagtgct acaaggtgag ctgcctggag 780

attcctgggc cccatggacc caagggctac cgaggacaga agggtgccaa gggcaacatg 840

ggtgagccag gagagcctgg acagaagggt cgacagggag accctggcat cgaaggcccc 900

attggattcc caggacccaa gggtgtacct ggcttcaagg gagagaaggg tgaatttgga 960

tccgatggtc ggaagggagc cccgggccta gctggcaaga atggaactga tggacagaag 1020

ggcaaactgg gccgcattgg gcctcctggt tgcaagggag acccaggaag tcggggcccc 1080

gatggatacc ctggagaagc cggaaccccg ggcgagcaag gagaccaagg tgccaagggg 1140

gactctggcc gcccaggacg caggggacca ccaggagatc ctggtgacaa gggaagcaag 1200

ggatatcaag gcaacaacgg agctcctgga agtccgggag tgaaaggagg caagggaggg 1260

cctggcccac gtggaccaaa aggagagccg ggacgcaggg gagatcccgg aaccaagggc 1320

ggcccaggaa ctgatggtcc aaagggggag aagggagacc ctggtcctga gggacctcga 1380

ggcctggctg gagaagttgg cagcaaagga gccaagggag acagaggttt gcctggaccc 1440

agaggccccc agggggctct tggggagctg ggaaaacagg gatctagggg agaccctggt 1500

gatgctggac cccgtggaga ttcaggacag ccaggaccca agggagatcc tggaaggcct 1560

ggattcagct acccaggacc ccgaggaaca cctggtgaaa aaggagagcc cggtccacca 1620

ggccctgagg gaggccgagg agactttggt ctgaaaggag cacctggaag gaagggagac 1680

aagggagaac cagctgatcc tggtccccct ggtgaacctg gccctcgggg cccaagagga 1740

atcccaggac ctgagggaga acccggccct cctggagacc ctggtctcac ggaatgtgac 1800

gtcatgacct atgtgaggga aacctgtggg tgctgcgact gtgagaagcg ttgtggtgct 1860

ctggatgtgg tcttcgtcat cgatagctct gagagtatcg gctacaccaa cttcaccttg 1920

gagaagaact ttgtcatcaa tgtggtcaac aggctgggtg ccattgccaa ggaccccaag 1980

tctgagacag gcacacgtgt aggtgtggtg cagtacagcc acgaaggcac ttttgaagcc 2040

atccggctag acgacgaacg agtcaactcc ctgtccagtt tcaaggaggc tgtcaaaaac 2100

ctcgagtgga ttgctggcgg cacctggaca ccctctgccc tcaagtttgc ctacaatcag 2160

ctcatcaaag agagccggcg ccagaagact cgggtgtttg cagtggtcat cacagatggg 2220

cgccatgacc cccgagatga cgacctcaat cttcgggcgc tgtgtgatcg agatgtcact 2280

gtgacggcca ttggcattgg tgacatgttc catgagacgc atgagagtga gaacctctac 2340

tccattgcct gtgacaagcc acagcaggtg cgcaatatga cacttttctc tgacctggtg 2400

gctgagaagt tcatcgatga catggaagat gtcctttgcc cagaccccca gatcgtgtgt 2460

ccagaacttc cctgccaaac agagctatat gtggcccagt gcacacagcg gcctgtggac 2520

attgtcttcc tgctggatgg ctcggaacgg ttgggtgagc agaacttcca caaggcacgg 2580

cgcttcgtgg aggaggtgtc acggcgcctg actctggcac gaaaggatga cgacccactc 2640

aacgcccgca tggccctgtt gcagtacggc agccagaatc agcagcaggt ggtcttccca 2700

ctgacctaca acttgaccac catccatgag gcactggaga ggaccgccta cctcaattcc 2760

ttttctcacg tgggtgcagg catcgtgcat gccatcaaca acgtggttcg gggcgcacgg 2820

ggcggggcac ggcgccacgc agagctctcc tttgtcttcc tcaccgatgg cgtcacgggc 2880

aatgacagcc tggaggagtc ggtgcactcc atgcgcaagc agaacgtggt gcccaccgtg 2940

gtcgctgtgg gcagcgatgt ggacatggat gtgcttacca agatcagcct gggtgacagg 3000

gcggccatct tccgggagaa agactttgac agtctggccc agcccagctt cttcgacagg 3060

ttcatccgtt ggatctgtta g 3081

<210> 17
<211> 207
<212> PRT
<213> Hamster

<400> 17

Met Ala Val Leu Arg Ser Leu Leu Pro Gln Leu Gly Leu Phe Leu Cys
1 5 10 15

```
Leu Ala Leu Cys Phe Ser Pro Ala Leu Ser Ala Ser Tyr Asn Asp Pro
            20                  25                  30

Cys Met Val Phe Asp Thr Ile Ser Thr Ser Asp Asn Leu Gly Val Ser
        35                  40                  45

Ile Thr Val Ala Gly Ser Ser Gly Glu Asn Ile Thr Tyr Thr Val Leu
    50                  55                  60

Val His Val Asn Ser Ser Val Ser Ala Val Ile Leu Lys Ala Val Asn
65                  70                  75                  80

Gln Asn Lys Pro Val Gly Ser Trp Val Gly Ala Thr Gln Glu Cys Asn
                85                  90                  95

Asp Ser Ser Val Leu Tyr Arg Val Thr Pro Ser Asp Ser Ser Gly Phe
            100                 105                 110

Gln Ala Thr Trp Ile Val Pro Asn Ser Glu Asp Met Thr Lys Val Asn
        115                 120                 125

Leu His Val Phe Leu Ala Thr Gly Asn Gly Thr Ala Ala Met Thr Ser
    130                 135                 140

Val Asn Leu Gly Glu Gln Lys Thr Pro Val Thr Val Asn Pro Thr Pro
145                 150                 155                 160

Glu Ile Ser Glu Thr Asn Gln Thr Thr Ala Met Thr Thr Asp Arg Thr
                165                 170                 175

Thr Ala Lys Ser Leu Ala Val Asn Ala Leu Gly Ser Pro Leu Ala Gly
            180                 185                 190

Ala Phe His Ile Leu Leu Val Phe Leu Ile Ser Lys Leu Leu Phe
        195                 200                 205

<210>  18
<211>  623
<212>  PRT
<213>  Hamster

<400>  18

Met Arg Gly Leu Phe Trp Pro Leu Ser Leu Leu Leu Leu Ser Leu Gln
1               5                   10                  15

Pro Trp Glu Ile Gln Leu Gln Ser Ala Gly Pro Arg Cys Tyr Thr Gly
            20                  25                  30
```

Pro Leu Asp Leu Val Phe Val Ile Asp Ser Ser Arg Ser Val Arg Pro
35 40 45

Phe Glu Phe Glu Thr Met Arg Gln Phe Leu Val Gly Leu Leu Arg Ser
50 55 60

Leu Asp Val Gly Leu Asn Ala Thr Arg Val Gly Val Ile Gln Tyr Ser
65 70 75 80

Ser Gln Val Gln Ser Val Phe Pro Leu Gly Ala Phe Ser Arg Arg Glu
85 90 95

Asp Met Glu Arg Ala Ile Ser Ala Val Val Pro Leu Ala Gln Gly Thr
100 105 110

Met Thr Gly Leu Ala Ile Gln Tyr Ala Met Asn Val Ala Phe Ser Glu
115 120 125

Ala Glu Gly Ala Arg Pro Pro Glu Glu Arg Val Pro Arg Val Leu Val
130 135 140

Ile Val Thr Asp Gly Arg Pro Gln Asp Arg Val Ala Glu Val Ala Ala
145 150 155 160

Gln Ala Arg Ala Arg Gly Ile Glu Ile Tyr Ala Val Gly Val Gln Arg
165 170 175

Ala Asp Val Gly Ser Leu Arg Ala Met Ala Ser Pro Pro Leu Asp Gln
180 185 190

His Val Phe Leu Val Glu Ser Phe Asp Leu Ile Gln Glu Phe Gly Leu
195 200 205

Gln Phe Gln Gly Arg Leu Cys Gly Lys Asp Leu Cys Ala Glu Trp Gly
210 215 220

His Gly Cys Gln His Gln Cys Val Asn Ala Pro Gly Thr Phe Tyr Cys
225 230 235 240

Thr Cys Asn Ser Gly Tyr Lys Leu Ala Pro Asp Asn Lys Asn Cys Leu
245 250 255

Ala Met Asp Leu Cys Ala Glu Gly Thr His Gly Cys Glu His Leu Cys
260 265 270

Val Asn Ser Leu Asp Ser Tyr Phe Cys Arg Cys Arg Ala Gly Phe Ala
275 280 285

```
Leu Gln Gln Asp Gln Lys Ser Cys Arg Ala Ile Asp Tyr Cys Ser Phe
    290                 295                 300

Gly Asn His Ser Cys Gln His Glu Cys Val Asn Thr Leu Glu Gly Pro
305                 310                 315                 320

Gln Cys Ile Cys Arg Glu Gly His Asp Leu Leu Pro Asp Gly Arg Ser
                325                 330                 335

Cys Arg Val Arg Asp Phe Cys Asn Gly Val Asp His Gly Cys Glu Phe
            340                 345                 350

Gln Cys Val Ser Glu Gly Leu Ser Phe Arg Cys Leu Cys Pro Glu Gly
        355                 360                 365

Arg Arg Leu Gln Ala Asp Gly Lys Ser Cys Asp Arg Cys Arg Glu Gly
    370                 375                 380

His Val Asp Leu Val Leu Leu Val Asp Gly Ser Lys Ser Val Arg Pro
385                 390                 395                 400

Gln Asn Phe Glu Leu Val Lys Arg Phe Val Asn Gln Ile Val Asp Phe
                405                 410                 415

Leu Asp Val Ser Pro Glu Gly Thr Arg Ile Gly Leu Val Gln Phe Ser
            420                 425                 430

Ser Arg Val Arg Thr Glu Phe Pro Leu Gly Arg Tyr Gly Thr Ala Ala
        435                 440                 445

Glu Val Lys Gln Ala Val Leu Ala Val Glu Tyr Met Glu Arg Gly Thr
    450                 455                 460

Met Thr Gly Leu Ala Leu Arg His Met Val Glu His Ser Phe Ser Glu
465                 470                 475                 480

Ala Gln Gly Ala Arg Pro Arg Asp Leu Asn Val Pro Arg Val Gly Leu
                485                 490                 495

Val Phe Thr Asp Gly Arg Ser Gln Asp Asp Ile Ser Val Trp Ala Ala
            500                 505                 510

Arg Ala Lys Glu Glu Gly Ile Val Met Tyr Ala Val Gly Val Gly Lys
        515                 520                 525

Ala Val Glu Glu Glu Leu Arg Glu Ile Ala Ser Glu Pro Ser Glu Leu
    530                 535                 540
```

His Val Ser Tyr Ala Pro Asp Phe Ser Thr Met Thr His Leu Leu Glu
545 550 555 560

Asn Leu Lys Gly Ser Ile Cys Pro Glu Glu Gly Ile Ser Ala Gly Thr
565 570 575

Glu Leu Arg Ser Pro Cys Glu Cys Glu Ser Leu Val Glu Phe Gln Gly
580 585 590

Arg Thr Leu Gly Ala Leu Glu Ser Leu Thr Arg Asn Leu Ala Gln Leu
595 600 605

Thr Glu Arg Leu Glu Glu Leu Glu Asn Gln Leu Thr Ser Arg Lys
610 615 620

<210> 19
<211> 903
<212> PRT
<213> Hamster

<400> 19

Met Arg Asp Leu Pro Thr Leu Pro Cys His Trp Val Cys Leu Leu Trp
1 5 10 15

Ser Phe Cys Ser Phe Gln Val Cys Gly Thr Gln Pro Arg Ala Gln Glu
20 25 30

His Pro Gly Phe Ala Val Leu Ala Ser Ala Ser His Tyr Trp Pro Leu
35 40 45

Glu Asn Val Asp Gly Ile Leu Glu Leu Gln Asp Thr Thr Gly Ala Leu
50 55 60

Arg Thr His Asn Leu Thr Val Pro Pro Ser His Asn Ala Thr Phe Val
65 70 75 80

Phe Thr Asn Asp Ser Ala Tyr Ser Asn Phe Ser Ala Thr Val Asp Ile
85 90 95

Met Glu Gly Lys Val Asn Lys Gly Ile Tyr Leu Lys Glu Glu Lys Gly
100 105 110

Val Thr Phe Leu Tyr Tyr Gly Ser Tyr Lys Ser Ser Cys Ile Ser Asn
115 120 125

Pro Ala Gln Cys Gly Pro Glu Gly Val Thr Phe Ser Phe Phe Trp Lys
130 135 140

Thr Gln Glu Glu Gln Pro Arg Pro Ala Pro Tyr Ala Tyr Gly Gly Gln
145 150 155 160

Val Ile Ser Asp Gly Phe Lys Val Cys Ser Ser Gly Gly Lys Gly Ser
165 170 175

Val Glu Leu Tyr Thr Arg Asp Asn Ser Met Thr Trp Lys Ala Thr Phe
180 185 190

Asn Pro Pro Gly Pro Tyr Trp Thr His Val Leu Phe Thr Trp Lys Ser
195 200 205

Lys Glu Gly Leu Lys Val Tyr Val Asn Gly Thr Leu Ser Thr Ser Asp
210 215 220

Pro Ser Gly Lys Val Ser His Thr Tyr Gly Glu Pro His Val Asn Leu
225 230 235 240

Val Ile Gly Ser Glu Gln Asp Gln Thr Lys Arg Tyr Glu Asn Gly Ala
245 250 255

Phe Asp Glu Phe Ile Ile Trp Glu Arg Ala Leu Thr Pro Asp Glu Ile
260 265 270

Lys Met Tyr Phe Thr Ala Ala Ile Gly Lys His Ser Leu Leu Ser Ser
275 280 285

Thr Pro Pro Ala Met Pro Thr Ala His Ser Met Val Pro Thr Asp Ala
290 295 300

Tyr His Pro Ile Ile Thr Asn Leu Thr Glu Glu Arg Lys Cys Phe Gln
305 310 315 320

Arg Pro Glu Thr Val Leu Arg Tyr Leu Gln Asn Val Ser Leu Ser Leu
325 330 335

Pro Asn Lys Ser Leu Ser Glu Glu Thr Ala Leu Asn Leu Thr Gln Thr
340 345 350

Phe Leu Arg Thr Val Gly Glu Val Leu Leu Leu Pro Ser Trp Thr His
355 360 365

Val Ser Glu Asp Asn Thr Met Thr Leu Gly Leu Val Asp Thr Ile Asp
370 375 380

Thr Val Met Gly His Ile Ser Ala Asn Leu Gln Ser Arg Glu Pro His

385 390 395 400

Ile Thr Leu Thr Gly Ser Ser Ser Met Ala Asp Phe Phe Val Ala Lys
405 410 415

Val Leu Pro Pro Ala Leu Ser Ala Pro His Tyr Arg Phe Pro Ala His
420 425 430

Gly His Ser Tyr Ile Glu Ile Pro Arg Glu Ala Leu His Gly Lys Ala
435 440 445

Trp Thr Thr Ile Val Gly Leu Leu Tyr His Ser Met His Tyr Tyr Leu
450 455 460

Asn Asn Ile Pro Pro Ala Ser Thr Lys Ile Pro Glu Ala Val Asn Cys
465 470 475 480

Arg Asp Cys Leu Leu Ser Val Ala Ser His Leu Ile Ser Leu Glu Val
485 490 495

Ser Pro Pro Pro Thr Leu Ser Gln Asn Leu Ser Gly Ser Pro Leu Ile
500 505 510

Thr Val His Leu Arg His Lys Leu Thr His Lys Gln Tyr Val Asp Ala
515 520 525

Thr Asn Glu Ser Asn Asp Leu Phe Leu Tyr Cys Ala Phe Leu Asn Phe
530 535 540

Ser Ser Gly Glu Gly Ile Trp Ser Ser Gln Gly Cys Ala Leu Arg Glu
545 550 555 560

Gly Asn Leu Thr His Ser Val Cys His Cys Thr His Leu Thr Asn Phe
565 570 575

Ala Ile Leu Met Gln Val Val Pro Leu Gln Leu Thr Arg Gly His Gln
580 585 590

Val Ala Leu Ser Ser Ile Ser Tyr Val Gly Cys Ser Leu Ser Val Leu
595 600 605

Cys Leu Ala Ala Thr Leu Val Thr Phe Ala Val Leu Ser Ser Val Ser
610 615 620

Thr Ile Arg Asn Gln Arg Tyr His Ile His Ala Asn Leu Ser Phe Ala
625 630 635 640

```
Val Leu Val Ala Gln Val Leu Leu Leu Ile Ser Phe Arg Val Glu Pro
                645                 650                 655

Gly Thr Val Pro Cys Gln Val Leu Ala Ile Leu Leu His Tyr Phe Phe
            660                 665                 670

Leu Ser Ala Phe Ala Trp Met Leu Val Glu Gly Leu His Leu Tyr Ser
        675                 680                 685

Met Val Ile Lys Val Phe Gly Ser Glu Asp Ser Lys His Leu Tyr Tyr
    690                 695                 700

Tyr Gly Ile Gly Trp Gly Cys Pro Leu Leu Ile Cys Ile Ile Ser Ile
705                 710                 715                 720

Ser Ser Ser Met Asp Ser Tyr Gly Thr Asn Asp Ser Cys Trp Leu Ser
                725                 730                 735

Leu Gly Ser Gly Ala Ile Trp Ala Phe Val Gly Pro Ala Leu Leu Val
            740                 745                 750

Ile Val Val Asn Ile Val Ile Leu Val Ala Val Thr Arg Val Ile Ser
        755                 760                 765

His Val Ser Thr Asp Asn Tyr Lys Ile His Gly Asp Pro Ser Ala Phe
    770                 775                 780

Lys Leu Thr Ala Lys Ala Val Ala Val Leu Leu Pro Ile Leu Gly Thr
785                 790                 795                 800

Ser Trp Val Phe Gly Val Leu Ala Val Ser Asp Arg Ala Leu Val Phe
                805                 810                 815

Gln Tyr Met Phe Ala Val Leu Asn Ser Leu Gln Gly Leu Phe Ile Phe
            820                 825                 830

Leu Phe His Cys Leu Leu Asn Ser Glu Val Arg Ala Ala Phe Lys His
        835                 840                 845

Lys Thr Lys Val Trp Ser Leu Thr Ser Ser Ser Ala Arg Thr Thr Asn
    850                 855                 860

Thr Lys Pro Phe Ser Ser Asp Ile Val Asn Gly Thr Arg Pro Gly Thr
865                 870                 875                 880

Ala Ser Thr Lys Leu Ser Pro Trp Asp Lys Ser Ser His Ser Ala His
                885                 890                 895
```

Arg Val Asp Leu Ser Ala Val
900

<210> 20
<211> 2019
<212> PRT
<213> Hamster

<400> 20

Met Gly Ala Met Thr Trp Leu Leu Pro Gly Ile Phe Leu Ala Leu Leu
1 5 10 15

Ala Leu Asn Ser Glu Gly Ala Val Leu Lys Lys Val Ile Arg His Lys
20 25 30

Arg Gln Ser Gly Leu Asn Met Thr Leu Pro Glu Glu Asn Gln Pro Val
35 40 45

Val Phe Asn His Val Tyr Asn Ile Lys Leu Pro Val Gly Ser Gln Cys
50 55 60

Ser Val Asp Leu Glu Ser Val Asn Gly Glu Lys Asp Leu Thr Pro Thr
65 70 75 80

Pro Glu Ser Ser Gly Thr Phe Gln Glu His Thr Val Asp Gly Glu Asn
85 90 95

Gln Ile Val Phe Thr His Arg Ile Asn Ile Pro Arg Arg Ala Cys Gly
100 105 110

Cys Ala Ala Ala Pro Asp Val Lys Glu Leu Leu Ser Arg Leu Glu Glu
115 120 125

Leu Glu Met Leu Val Ser Ser Leu Arg Glu Gln Cys Thr Met Gly Thr
130 135 140

Gly Cys Cys Leu Gln Pro Ala Glu Gly Arg Leu Asp Thr Arg Pro Phe
145 150 155 160

Cys Ser Gly Arg Gly Asn Phe Ser Ala Glu Gly Cys Gly Cys Val Cys
165 170 175

Glu Pro Gly Trp Lys Gly Pro Asn Cys Ser Glu Pro Glu Cys Pro Gly
180 185 190

Asn Cys Asn Leu Gln Gly Gln Cys Leu Asp Gly Gln Cys Val Cys Asp
195 200 205

Glu Gly Phe Thr Gly Glu Asp Cys Ser Gln Leu Ala Cys Pro Asn Asp
    210                 215                 220

Cys Asn Asp Gln Gly Arg Cys Val Asn Gly Val Cys Val Cys Phe Glu
225                 230                 235                 240

Gly Tyr Ala Gly Val Asp Cys Gly Leu Glu Val Cys Pro Val Pro Cys
                245                 250                 255

Ser Glu Glu His Gly Thr Cys Leu Asp Gly Arg Cys Val Cys Lys Asp
            260                 265                 270

Gly Phe Ala Gly Asp Asp Cys Asn Glu Pro Leu Cys Leu Asn Asn Cys
        275                 280                 285

Tyr Asn Arg Gly Arg Cys Val Glu Asn Glu Cys Val Cys Asp Glu Gly
    290                 295                 300

Phe Thr Gly Glu Asp Cys Ser Glu Leu Ile Cys Pro Asn Asp Cys Phe
305                 310                 315                 320

Asp Arg Gly Arg Cys Val Asn Gly Thr Cys Tyr Cys Glu Glu Gly Phe
                325                 330                 335

Thr Gly Glu Asp Cys Gly Glu Leu Thr Cys Pro Asn Asn Cys Gln Gly
            340                 345                 350

His Gly Gln Cys Glu Glu Gly Gln Cys Val Cys Asp Glu Gly Phe Ala
        355                 360                 365

Gly Ala Asp Cys Ser Glu Lys Arg Cys Pro Glu Asp Cys His His Arg
    370                 375                 380

Gly Arg Cys Leu Asn Gly Gln Cys Glu Cys Asp Asp Gly Phe Met Gly
385                 390                 395                 400

Ala Asp Cys Gly Asp Leu Gln Cys Pro Asn Gly Cys Ser Gly His Gly
                405                 410                 415

Arg Cys Val Asn Gly Gln Cys Val Cys Asp Glu Gly Tyr Thr Gly Glu
            420                 425                 430

Asp Cys Ser Gln Gln Arg Cys Pro Asn Asp Cys His Asn Arg Gly Leu
        435                 440                 445

Cys Val Gln Gly Lys Cys Ile Cys Glu Gln Gly Phe Lys Gly Phe Asp
    450                 455                 460

```
Cys Ser Glu Met Ser Cys Pro Asn Asp Cys His Gly His Gly Arg Cys
465                 470                 475                 480

Val Asn Gly Met Cys Ile Cys Asp Asp Glu Tyr Thr Gly Glu Asp Cys
                485                 490                 495

Arg Asp His Arg Cys Pro Arg Asp Cys Ser Gln Arg Gly Arg Cys Leu
            500                 505                 510

Asp Gly Gln Cys Ile Cys Glu Asp Gly Phe Thr Gly Pro Asp Cys Ala
        515                 520                 525

Glu Leu Ser Cys Pro Asn Asp Cys His Gly His Gly Arg Cys Val Asn
    530                 535                 540

Gly Gln Cys Ile Cys His Glu Gly Phe Thr Gly Lys Asp Cys Lys Glu
545                 550                 555                 560

Gln Arg Cys Pro Ser Asp Cys His Gly Gln Gly Arg Cys Glu Asp Gly
                565                 570                 575

Gln Cys Ile Cys His Glu Gly Phe Thr Gly Leu Asp Cys Gly Gln Arg
            580                 585                 590

Ser Cys Pro Asn Asp Cys Ser Asn Gln Gly Gln Cys Val Ser Gly Arg
        595                 600                 605

Cys Ile Cys Asn Glu Gly Tyr Arg Gly Glu Asp Cys Ser Glu Val Ser
    610                 615                 620

Pro Pro Lys Asp Leu Ile Val Thr Glu Val Thr Glu Glu Thr Val Asn
625                 630                 635                 640

Leu Ala Trp Asp Asn Glu Met Arg Val Thr Glu Tyr Leu Ile Met Tyr
                645                 650                 655

Thr Pro Thr His Ala Asp Gly Leu Glu Met Gln Phe Arg Val Pro Gly
            660                 665                 670

Asp Gln Thr Ser Thr Thr Ile Arg Glu Leu Glu Pro Gly Val Glu Tyr
        675                 680                 685

Phe Ile Arg Val Phe Ala Ile Leu Glu Asn Lys Arg Ser Ile Pro Val
    690                 695                 700

Ser Ala Arg Val Ala Thr Tyr Leu Pro Ala Pro Glu Gly Leu Lys Phe
```

705 710 715 720

Lys Ser Ile Lys Glu Thr Ser Val Glu Val Glu Trp Asp Pro Leu Asp
725 730 735

Ile Ala Phe Glu Thr Trp Glu Ile Ile Phe Arg Asn Met Asn Lys Glu
740 745 750

Asp Glu Gly Glu Ile Thr Lys Ser Leu Arg Arg Pro Glu Thr Ser Tyr
755 760 765

Arg Gln Thr Gly Leu Ala Pro Gly Gln Glu Tyr Glu Ile Ser Leu His
770 775 780

Ile Val Lys Asn Asn Thr Arg Gly Pro Gly Leu Lys Lys Val Thr Thr
785 790 795 800

Thr Arg Leu Asp Ala Pro Ser Gln Ile Glu Val Arg Asp Val Thr Asp
805 810 815

Thr Thr Ala Leu Ile Thr Trp Phe Lys Pro Leu Ala Glu Ile Asp Gly
820 825 830

Ile Glu Leu Ser Tyr Gly Ile Lys Asp Val Pro Gly Asp Arg Thr Thr
835 840 845

Ile Asp Leu Thr His Glu Glu Asn Gln Tyr Ser Ile Gly Asn Leu Lys
850 855 860

Pro Asp Thr Glu Tyr Glu Val Ser Leu Val Ser Arg Arg Val Asp Met
865 870 875 880

Ala Ser Asn Pro Ala Lys Glu Thr Phe Thr Thr Gly Leu Asp Ala Pro
885 890 895

Arg Asn Leu Arg Arg Val Ser Gln Thr Asp Asn Ser Ile Thr Leu Glu
900 905 910

Trp Arg Asn Val Lys Ala Asp Ile Asp Ser Tyr Arg Ile Lys Tyr Ala
915 920 925

Pro Ile Ser Gly Gly Asp His Ala Glu Val Asp Val Pro Lys Ser Gln
930 935 940

Gln Ala Thr Thr Lys Thr Thr Leu Thr Gly Leu Arg Pro Gly Thr Glu
945 950 955 960

Tyr Gly Val Gly Val Ser Ala Val Lys Gly Asp Lys Glu Ser Asp Pro
965 970 975

Ala Thr Ile Asn Ala Ala Thr Glu Ile Asp Ala Pro Arg Asp Phe Gln
980 985 990

Val Ser Glu Thr Thr Gln Asp Ser Leu Thr Leu Leu Trp Lys Thr Pro
995 1000 1005

Leu Ala Lys Phe Asp Arg Tyr Arg Leu Asn Tyr Ser Leu Pro Thr
1010 1015 1020

Gly Gln Ser Ile Glu Val Gln Leu Pro Lys Asp Ala Thr Ser His
1025 1030 1035

Val Leu Thr Asp Leu Glu Pro Gly Lys Glu Tyr Thr Val Leu Leu
1040 1045 1050

Thr Ala Glu Lys Gly Arg His Lys Ser Lys Pro Ala Arg Val Lys
1055 1060 1065

Ala Ser Thr Glu Gln Ala Pro Ser Leu Glu Asn Leu Thr Val Thr
1070 1075 1080

Glu Ala Gly Trp Asp Gly Leu Arg Leu Asn Trp Thr Ala Asp Asp
1085 1090 1095

Leu Ala Tyr Glu Tyr Phe Val Ile Gln Val Gln Glu Ala Asn Lys
1100 1105 1110

Val Glu Ala Ala His Asn Phe Thr Val Leu Gly Asn Leu Arg Ala
1115 1120 1125

Thr Asp Ile Pro Gly Leu Lys Ala Ala Thr Pro Tyr Arg Val Ser
1130 1135 1140

Ile Tyr Gly Val Ala Arg Gly Ser Arg Thr Pro Val Leu Ser Ala
1145 1150 1155

Glu Ala Ser Thr Gly Lys Thr Pro Asn Leu Gly Glu Val Ser Val
1160 1165 1170

Ala Glu Val Gly Trp Asp Ala Leu Lys Leu Asn Trp Thr Ala Pro
1175 1180 1185

Glu Gly Val Tyr Gln Asn Phe Phe Ile Gln Val Leu Glu Ala Asp
1190 1195 1200

Thr Thr Gln Thr Val Gln Asn Leu Thr Val Pro Gly Gly Leu Arg
1205 1210 1215

Ser Val Asp Leu Pro Gly Leu Arg Ala Ala Thr His Tyr His Ile
1220 1225 1230

Thr Ile Arg Gly Val Thr Gln Asp Phe Ser Thr Ala Pro Leu Ser
1235 1240 1245

Val Glu Val Leu Thr Glu Glu Leu Pro His Leu Gly Gly Leu Ser
1250 1255 1260

Val Thr Glu Val Ser Trp Asp Gly Leu Ile Leu Asn Trp Thr Thr
1265 1270 1275

Asp Asp Leu Ala Tyr Glu His Phe Val Ile Gln Val Gln Glu Ala
1280 1285 1290

Asn Asn Val Glu Ala Ala Gln Asn Leu Thr Val Pro Gly Ser Ile
1295 1300 1305

Arg Ala Met Glu Ile Pro Gly Leu Lys Ala Ala Thr Pro Tyr Arg
1310 1315 1320

Val Ser Ile Tyr Gly Val Ile Gln Gly Tyr Arg Thr Pro Met Leu
1325 1330 1335

Ser Ala Asp Ala Ser Thr Ala Lys Glu Pro Glu Ile Gly Asn Leu
1340 1345 1350

Asn Val Ser Asp Val Thr Pro Glu Ser Phe Asn Leu Ser Trp Thr
1355 1360 1365

Ala Thr Asp Gly Ile Phe Asp Met Phe Thr Ile Glu Ile Ile Asp
1370 1375 1380

Ser Asn Arg Leu Leu Gln Met Ala Glu His Asn Ile Ser Gly Ala
1385 1390 1395

Glu Arg Thr Ala His Ile Ser Gly Leu Pro Pro Ser Thr Asp Phe
1400 1405 1410

Ile Val Tyr Leu Ser Gly Ile Ala Pro Ser Ile Arg Thr Lys Thr
1415 1420 1425

Ile Ser Thr Thr Ala Thr Thr Glu Ala Glu Pro Glu Val Asp Asn
1430 1435 1440

Leu Leu Val Ser Asp Ala Thr Pro Gly Gly Phe Arg Leu Ser Trp
1445 1450 1455

Thr Ala Asp Glu Gly Ile Phe Asp Ser Phe Val Leu Arg Ile Arg
1460 1465 1470

Asp Thr Lys Lys Gln Ser Glu Pro Gln Glu Ile Ile Leu Pro Ser
1475 1480 1485

Pro Glu Arg Thr Arg Asp Ile Thr Gly Leu Arg Glu Ala Thr Glu
1490 1495 1500

Tyr Glu Ile Glu Leu Tyr Gly Ile Ser Gly Gly Arg Arg Ser Gln
1505 1510 1515

Pro Val Ser Ala Ile Ala Thr Thr Ala Met Gly Ser Pro Lys Glu
1520 1525 1530

Ile Met Phe Ser Asp Ile Thr Asp Asn Ala Ala Thr Val Ser Trp
1535 1540 1545

Lys Ala Pro Thr Ala Gln Val Glu Ser Phe Arg Ile Thr Tyr Val
1550 1555 1560

Pro Met Thr Gly Gly Ala Pro Ser Met Val Thr Val Asp Gly Thr
1565 1570 1575

Asp Thr Glu Thr Arg Leu Val Lys Leu Thr Pro Gly Val Glu Tyr
1580 1585 1590

His Val Ser Val Ile Ala Met Lys Gly Phe Glu Glu Ser Asp Pro
1595 1600 1605

Val Ser Gly Ser Leu Thr Thr Ala Leu Asp Gly Pro Ser Gly Leu
1610 1615 1620

Leu Ala Ala Asn Ile Thr Asp Thr Glu Ala Leu Ala Leu Trp Gln
1625 1630 1635

Pro Ala Ile Ala Thr Val Asp Ser Tyr Val Ile Ser Tyr Thr Gly
1640 1645 1650

Glu Arg Val Pro Glu Val Thr Arg Thr Val Ser Gly Asn Thr Val
1655 1660 1665

Glu Tyr Glu Leu His Asp Leu Glu Pro Ala Thr Glu Tyr Thr Leu

1670 1675 1680

Arg Ile Phe Ala Glu Lys Gly His Gln Lys Ser Ser Ala Ile Thr
1685 1690 1695

Thr Lys Phe Thr Thr Asp Leu Asp Ser Pro Arg Asp Leu Thr Ala
1700 1705 1710

Thr Glu Val Gln Ser Glu Thr Ala Leu Leu Thr Trp Arg Pro Pro
1715 1720 1725

Arg Ala Ser Ile Thr Gly Tyr Leu Leu Val Tyr Glu Ser Leu Asp
1730 1735 1740

Gly Thr Val Lys Glu Val Ile Val Gly Pro Asp Thr Thr Ser Tyr
1745 1750 1755

Ser Leu Ala Asp Leu Ser Pro Ser Thr His Tyr Thr Val Arg Ile
1760 1765 1770

Gln Ala Leu Ser Gly Ser Leu Arg Ser Lys Leu Ile Gln Thr Ile
1775 1780 1785

Phe Thr Thr Ile Gly Leu Leu Tyr Pro Phe Pro Arg Asp Cys Ser
1790 1795 1800

Gln Ala Met Leu Asn Gly Asp Thr Thr Ser Gly Leu Tyr Thr Ile
1805 1810 1815

Tyr Ile Asn Gly Asp Lys Thr Gln Ala Leu Glu Val Tyr Cys Asp
1820 1825 1830

Met Thr Ser Asp Gly Gly Gly Trp Ile Val Phe Leu Arg Arg Lys
1835 1840 1845

Asn Gly Arg Glu Asp Phe Tyr Arg Asn Trp Lys Ala Tyr Ala Ala
1850 1855 1860

Gly Phe Gly Asp Arg Arg Glu Glu Phe Trp Leu Gly Leu Asp Asn
1865 1870 1875

Leu Ser Lys Ile Thr Ala Gln Gly Gln Tyr Glu Leu Arg Val Asp
1880 1885 1890

Leu Gln Asp His Gly Glu Ser Ala Phe Ala Val Tyr Asp Arg Phe
1895 1900 1905

Ser Val Gly Asp Ala Lys Ser Arg Tyr Lys Leu Lys Val Glu Gly
1910 1915 1920

Tyr Ser Gly Thr Ala Gly Asp Ser Met Asn Tyr His Asn Gly Arg
1925 1930 1935

Ser Phe Ser Thr Tyr Asp Lys Asp Thr Asp Ser Ala Ile Thr Asn
1940 1945 1950

Cys Ala Leu Ser Tyr Lys Gly Ala Phe Trp Tyr Lys Asn Cys His
1955 1960 1965

Arg Val Asn Leu Met Gly Arg Tyr Gly Asp Asn Asn His Ser Gln
1970 1975 1980

Gly Val Asn Trp Phe His Trp Lys Gly His Glu Tyr Ser Ile Gln
1985 1990 1995

Phe Ala Glu Met Lys Leu Arg Pro Ser Asn Phe Arg Asn Leu Glu
2000 2005 2010

Gly Arg Arg Lys Arg Ala
2015

<210> 21
<211> 1466
<212> PRT
<213> Hamster

<400> 21

Met Met Ser Phe Val Gln Ser Gly Thr Trp Leu Leu Leu Ala Leu Leu
1 5 10 15

His Pro Thr Phe Ile Trp Ala Gln Gln Ser Ser Val Asp Glu Ser Gly
20 25 30

Cys Ser His Leu Gly Gln Ser Tyr Glu Ser Arg Asp Val Trp Lys Pro
35 40 45

Glu Pro Cys Gln Ile Cys Val Cys Asp Ser Gly Ser Val Leu Cys Asp
50 55 60

Asp Ile Ile Cys Asp Glu Asp Pro Leu Asp Cys Pro Asn Pro Glu Ile
65 70 75 80

Pro Phe Gly Glu Cys Cys Ala Ile Cys Pro Gln Pro Ser Thr Ala Ala
85 90 95

Pro Val Pro Pro Asp Gly His Gly Pro Gln Gly Pro Lys Gly Asp Pro
100 105 110

Gly Pro Pro Gly Ile Pro Gly Arg Asn Gly Asp Pro Gly Leu Pro Gly
115 120 125

Gln Pro Gly Leu Pro Gly Pro Pro Gly Ser Pro Gly Ile Cys Glu Ser
130 135 140

Cys Pro Thr Gly Gly Gln Asn Tyr Ser Pro Gln Tyr Asp Ser Tyr Asp
145 150 155 160

Val Lys Ser Gly Val Gly Ala Gly Gly Leu Gly Gly Gly Tyr Pro Gly
165 170 175

Pro Ala Gly Pro Pro Gly Pro Pro Gly Pro Pro Gly Ser Val Gly His
180 185 190

Pro Gly Ser Pro Gly Ser Pro Gly Tyr Gln Gly Pro Pro Gly Glu Pro
195 200 205

Gly Gln Ala Gly Pro Ser Gly Pro Pro Gly Pro Pro Gly Ala Ile Gly
210 215 220

Pro Ser Gly Pro Ala Gly Lys Asp Gly Glu Ser Gly Arg Pro Gly Arg
225 230 235 240

Pro Gly Glu Arg Gly Leu Pro Gly Pro Pro Gly Ile Lys Gly Pro Ser
245 250 255

Gly Met Pro Gly Phe Pro Gly Met Lys Gly His Arg Gly Phe Asp Gly
260 265 270

Arg Asn Gly Glu Lys Gly Glu Thr Gly Ala Pro Gly Leu Lys Gly Glu
275 280 285

Asn Gly Leu Pro Gly Asp Asn Gly Ala Pro Gly Pro Met Gly Pro Arg
290 295 300

Gly Ala Pro Gly Glu Arg Gly Arg Pro Gly Leu Pro Gly Ala Ala Gly
305 310 315 320

Ala Arg Gly Asn Asp Gly Ala Arg Gly Ser Asp Gly Gln Pro Gly Pro
325 330 335

Pro Gly Pro Pro Gly Thr Ala Gly Phe Pro Gly Ser Pro Gly Ala Lys
340 345 350

Gly Glu Val Gly Pro Ala Gly Ser Pro Gly Ser Asn Gly Ser Pro Gly
355 360 365

Gln Arg Gly Glu Pro Gly Pro Gln Gly His Ala Gly Ala Gln Gly Pro
370 375 380

Pro Gly Pro Pro Gly Asn Asn Gly Ser Pro Gly Gly Lys Gly Glu Met
385 390 395 400

Gly Pro Ala Gly Ile Pro Gly Ala Pro Gly Leu Met Gly Ala Arg Gly
405 410 415

Pro Pro Gly Pro Ala Gly Thr Asn Gly Ala Pro Gly Gln Arg Gly Pro
420 425 430

Ser Gly Glu Pro Gly Lys Asn Gly Ala Lys Gly Glu Pro Gly Ala Arg
435 440 445

Gly Glu Arg Gly Glu Ala Gly Ser Pro Gly Ile Pro Gly Pro Lys Gly
450 455 460

Glu Asp Gly Lys Asp Gly Ser Pro Gly Glu Pro Gly Ala Asn Gly Leu
465 470 475 480

Pro Gly Thr Ala Gly Glu Arg Gly Ala Pro Gly Phe Arg Gly Pro Ala
485 490 495

Gly Pro Asn Gly Ile Pro Gly Glu Lys Gly Pro Ala Gly Glu Arg Gly
500 505 510

Ala Pro Gly Pro Ala Gly Pro Arg Gly Val Ala Gly Glu Pro Gly Arg
515 520 525

Asp Gly Asn Pro Gly Gly Pro Gly Met Arg Gly Val Pro Gly Ser Pro
530 535 540

Gly Gly Pro Gly Asn Asp Gly Lys Pro Gly Pro Pro Gly Ser Gln Gly
545 550 555 560

Glu Ser Gly Arg Pro Gly Pro Pro Gly Pro Ser Gly Pro Arg Gly Gln
565 570 575

Pro Gly Val Met Gly Phe Pro Gly Pro Lys Gly Asn Asp Gly Ala Pro
580 585 590

Gly Lys Asn Gly Glu Arg Gly Gly Pro Gly Gly Pro Gly Leu Pro Gly
595 600 605

```
Pro Ala Gly Lys Asn Gly Glu Thr Gly Pro Gln Gly Pro Pro Gly Pro
    610                 615                 620

Thr Gly Pro Ser Gly Asp Lys Gly Glu Ser Gly Pro Pro Gly Pro Gln
625                 630                 635                 640

Gly Leu Gln Gly Ile Pro Gly Thr Ser Gly Pro Pro Gly Glu Asn Gly
                645                 650                 655

Lys Pro Gly Glu Pro Gly Pro Lys Gly Glu Val Gly Ala Pro Gly Val
            660                 665                 670

Pro Gly Gly Lys Gly Asp Ala Gly Ala Pro Gly Glu Arg Gly Pro Pro
        675                 680                 685

Gly Thr Ala Gly Val Pro Gly Leu Arg Gly Gly Ala Gly Pro Pro Gly
    690                 695                 700

Pro Glu Gly Gly Lys Gly Pro Ala Gly Pro Pro Gly Pro Pro Gly Thr
705                 710                 715                 720

Ser Gly Ser Pro Gly Leu Gln Gly Met Pro Gly Glu Arg Gly Gly Pro
                725                 730                 735

Gly Ser Pro Gly Pro Lys Gly Glu Lys Gly Glu Pro Gly Gly Ala Gly
            740                 745                 750

Ala Asp Gly Ala Pro Gly Lys Asp Gly Pro Arg Gly Pro Thr Gly Pro
        755                 760                 765

Ile Gly Pro Pro Gly Pro Ala Gly Gln Pro Gly Asp Lys Gly Glu Gly
    770                 775                 780

Gly Ala Pro Gly Leu Pro Gly Ile Ala Gly Pro Arg Gly Gly Pro Gly
785                 790                 795                 800

Glu Arg Gly Glu His Gly Pro Pro Gly Pro Ala Gly Phe Pro Gly Ala
                805                 810                 815

Pro Gly Gln Asn Gly Glu Pro Gly Ala Lys Gly Glu Arg Gly Ala Pro
            820                 825                 830

Gly Glu Lys Gly Glu Gly Gly Pro Pro Gly Pro Ala Gly Leu Pro Gly
        835                 840                 845

Gly Ser Gly Pro Ala Gly Pro Pro Gly Pro Gln Gly Val Lys Gly Glu
```

850 855 860

Arg Gly Ser Pro Gly Gly Pro Gly Ala Ala Gly Phe Pro Gly Gly Arg
865 870 875 880

Gly Leu Pro Gly Pro Pro Gly Asn Asn Gly Asn Pro Gly Pro Pro Gly
885 890 895

Pro Ser Gly Ala Pro Gly Lys Asp Gly Pro Pro Gly Pro Ala Gly Asn
900 905 910

Ser Gly Ser Pro Gly Asn Pro Gly Val Ala Gly Pro Lys Gly Asp Ala
915 920 925

Gly Gln Pro Gly Glu Lys Gly Pro Pro Gly Ala Gln Gly Pro Pro Gly
930 935 940

Ser Pro Gly Pro Leu Gly Ile Ala Gly Leu Thr Gly Ala Arg Gly Leu
945 950 955 960

Ala Gly Pro Pro Gly Met Pro Gly Pro Arg Gly Ser Pro Gly Pro Gln
965 970 975

Gly Ile Lys Gly Glu Ser Gly Lys Pro Gly Ala Ser Gly His Asn Gly
980 985 990

Glu Arg Gly Pro Pro Gly Pro Gln Gly Leu Pro Gly Gln Pro Gly Thr
995 1000 1005

Ala Gly Glu Pro Gly Arg Asp Gly Asn Pro Gly Ser Asp Gly Gln
1010 1015 1020

Pro Gly Arg Asp Gly Ser Pro Gly Gly Lys Gly Asp Arg Gly Glu
1025 1030 1035

Asn Gly Ser Pro Gly Ala Pro Gly Ala Pro Gly His Pro Gly Pro
1040 1045 1050

Pro Gly Pro Val Gly Pro Ala Gly Lys Ser Gly Asp Arg Gly Glu
1055 1060 1065

Thr Gly Pro Ala Gly Pro Ser Gly Ala Pro Gly Pro Ala Gly Ser
1070 1075 1080

Arg Gly Pro Pro Gly Pro Gln Gly Pro Arg Gly Asp Lys Gly Glu
1085 1090 1095

Thr Gly Glu Arg Gly Ser Asn Gly Ile Lys Gly His Arg Gly Phe
1100 1105 1110

Pro Gly Asn Pro Gly Pro Pro Gly Ser Pro Gly Ala Ala Gly His
1115 1120 1125

Gln Gly Ala Val Gly Ser Pro Gly Pro Ala Gly Pro Arg Gly Pro
1130 1135 1140

Val Gly Pro His Gly Pro Pro Gly Lys Asp Gly Thr Ser Gly His
1145 1150 1155

Pro Gly Pro Ile Gly Pro Pro Gly Pro Arg Gly Asn Arg Gly Glu
1160 1165 1170

Arg Gly Ser Glu Gly Ser Pro Gly His Pro Gly Gln Pro Gly Pro
1175 1180 1185

Pro Gly Pro Pro Gly Ala Pro Gly Pro Cys Cys Gly Gly Gly Ala
1190 1195 1200

Ala Ala Leu Gly Gly Gly Gly Glu Lys Ser Gly Gly Phe Ser Pro
1205 1210 1215

Tyr Tyr Gly Asp Glu Pro Met Asp Phe Lys Ile Asn Thr Glu Glu
1220 1225 1230

Ile Met Ser Ser Leu Lys Ser Val Asn Gly Gln Ile Glu Ser Leu
1235 1240 1245

Ile Ser Pro Asp Gly Ser Arg Lys Asn Pro Ala Arg Asn Cys Arg
1250 1255 1260

Asp Leu Lys Phe Cys His Pro Asp Leu Lys Ser Gly Glu Tyr Trp
1265 1270 1275

Val Asp Pro Asn Gln Gly Cys Lys Met Asp Ala Ile Lys Val Phe
1280 1285 1290

Cys Asn Met Glu Thr Gly Glu Thr Cys Ile Asn Ala Ser Pro Met
1295 1300 1305

Thr Val Pro Arg Lys Asn Trp Trp Thr Asp Ala Ser Ala Glu Lys
1310 1315 1320

Lys His Val Trp Phe Gly Glu Ser Met Asn Gly Gly Phe Gln Phe
1325 1330 1335

Ser Tyr Gly Asn Pro Asp Leu Pro Glu Asp Val Leu Asp Val Gln
1340 1345 1350

Leu Ala Tyr Leu Arg Leu Leu Ser Ser Arg Ala Ser Gln Asn Ile
1355 1360 1365

Thr Tyr His Cys Lys Asn Ser Ile Ala Tyr Met Asp Gln Ala Ser
1370 1375 1380

Gly Asn Val Lys Lys Ser Leu Lys Leu Met Gly Ser Asn Glu Gly
1385 1390 1395

Glu Phe Lys Ala Glu Gly Asn Ser Lys Phe Thr Tyr Thr Val Leu
1400 1405 1410

Glu Asp Gly Cys Ala Lys His Thr Gly Glu Trp Ser Lys Thr Val
1415 1420 1425

Phe Glu Tyr Arg Thr Arg Lys Ala Leu Arg Leu Pro Ile Ile Asp
1430 1435 1440

Ile Ala Pro Tyr Asp Ile Gly Gly Pro Asp Gln Glu Phe Gly Val
1445 1450 1455

Asp Ile Gly Pro Val Cys Phe Leu
1460 1465

<210> 22
<211> 221
<212> PRT
<213> Hamster

<400> 22

Met Ala Gly Lys Pro Val Leu His Tyr Phe Asp Gly Gly Gly Arg Met
1 5 10 15

Glu Pro Val Arg Trp Leu Leu Ala Ala Ala Gly Val Glu Phe Glu Glu
20 25 30

Lys Phe Leu Lys Thr Arg Asp Asp Leu Ala Arg Leu Arg Asn Asp Gly
35 40 45

Ser Leu Met Phe Gln Gln Val Pro Met Val Glu Ile Asp Gly Met Lys
50 55 60

Leu Val Gln Thr Arg Ala Ile Leu Asn Tyr Ile Ala Ser Lys Tyr Asn
65 70 75 80

```
Leu Tyr Gly Lys Asp Met Lys Glu Arg Ala Leu Ile Asp Met Tyr Ala
                85                  90                  95

Glu Gly Ile Ala Asp Leu Asp Glu Ile Val Leu His Gln Pro Tyr Ile
            100                 105                 110

Pro Gln Glu Glu Lys Glu Ala Asn Leu Ala Lys Ile Lys Asp Lys Ala
        115                 120                 125

Arg Asn Arg Tyr Phe Pro Ala Tyr Glu Lys Val Leu Lys Gly His Gly
    130                 135                 140

Gln Asp Tyr Leu Val Gly Asn Arg Leu Ser Arg Ala Asp Val Tyr Leu
145                 150                 155                 160

Val Glu Leu Leu Tyr His Val Glu Glu Leu Asp Pro Ser Val Leu Ala
                165                 170                 175

Asn Phe Pro Leu Leu Lys Ala Leu Arg Thr Arg Val Ser Asn Leu Pro
            180                 185                 190

Thr Val Lys Lys Phe Leu Gln Pro Gly Ser Gln Arg Lys Pro Tyr Glu
        195                 200                 205

Asp Glu Lys Cys Val Glu Ser Ala Met Lys Ile Phe Ser
    210                 215                 220


<210>  23
<211>  607
<212>  PRT
<213>  Hamster

<400>  23

Met Trp Leu Arg Ser Leu Val Lys Gln Leu Glu Arg Gly Glu Ala Ser
1               5                   10                  15

Val Val Asp Leu Lys Lys Asn Leu Glu Tyr Ala Ala Thr Val Leu Glu
            20                  25                  30

Ser Val Tyr Ile Asp Glu Thr Arg Arg Leu Leu Asp Thr Glu Asp Glu
        35                  40                  45

Leu Ser Asp Ile Gln Ser Asp Ala Val Pro Ser Glu Val Arg Asp Trp
    50                  55                  60

Leu Ala Ser Thr Phe Thr Arg Gln Met Gly Met Met Leu Arg Arg Ser
65                  70                  75                  80
```

```
Asp Glu Lys Pro Arg Phe Lys Ser Ile Val His Ala Val Gln Ala Gly
                85                  90                  95

Ile Phe Val Glu Arg Met Tyr Arg Arg Thr Ser Asn Met Val Gly Leu
            100                 105                 110

Ser Tyr Pro Pro Ala Val Ile Asp Ala Leu Lys Asp Val Asp Lys Trp
        115                 120                 125

Ser Phe Asp Val Phe Ser Leu Asn Asp Ala Ser Gly Asp His Ala Leu
    130                 135                 140

Lys Phe Ile Phe Tyr Glu Leu Leu Thr Arg Tyr Asp Leu Ile Ser Arg
145                 150                 155                 160

Phe Lys Ile Pro Ile Ser Ala Leu Val Ser Phe Val Glu Ala Leu Glu
                165                 170                 175

Val Gly Tyr Ser Lys His Lys Asn Pro Tyr His Asn Leu Met His Ala
            180                 185                 190

Ala Asp Val Thr Gln Thr Val His Tyr Leu Leu Tyr Lys Thr Gly Val
        195                 200                 205

Ala Asn Trp Leu Thr Glu Leu Glu Ile Phe Ala Ile Ile Phe Ser Ala
    210                 215                 220

Ala Ile His Asp Tyr Glu His Thr Gly Thr Thr Asn Asn Phe His Ile
225                 230                 235                 240

Gln Thr Arg Ser Asp Pro Ala Ile Leu Tyr Asn Asp Arg Ser Val Leu
                245                 250                 255

Glu Asn His His Leu Ser Ala Ala Tyr Arg Leu Leu Gln Glu Asp Glu
            260                 265                 270

Glu Met Asn Ile Leu Val Asn Leu Ser Lys Asp Asp Trp Arg Glu Phe
        275                 280                 285

Arg Thr Leu Val Ile Glu Met Val Met Ala Thr Asp Met Ser Cys His
    290                 295                 300

Phe Gln Gln Ile Lys Ala Met Lys Thr Ala Leu Gln Gln Pro Glu Ala
305                 310                 315                 320

Ile Glu Lys Pro Lys Ala Leu Ser Leu Met Leu His Thr Ala Asp Ile
                325                 330                 335
```

```
Ser His Pro Ala Lys Ala Trp Asp Leu His His Arg Trp Thr Met Ser
            340                 345                 350

Leu Leu Glu Glu Phe Phe Arg Gln Gly Asp Arg Glu Ala Glu Leu Gly
        355                 360                 365

Leu Pro Phe Ser Pro Leu Cys Asp Arg Lys Ser Thr Met Val Ala Gln
    370                 375                 380

Ser Gln Val Gly Phe Ile Asp Phe Ile Val Glu Pro Thr Phe Thr Val
385                 390                 395                 400

Leu Thr Asp Met Thr Glu Lys Ile Val Ser Pro Leu Ile Asp Glu Thr
                405                 410                 415

Ser Gln Thr Gly Gly Thr Gly Gln Arg Arg Ser Ser Leu Asn Asn Ile
            420                 425                 430

Ser Ala Ser Asp Ala Lys Arg Pro Gly Val Lys Ser Ser Gly Ser Glu
        435                 440                 445

Gly Ser Ala Pro Ile Asn Asn Ser Val Ile Pro Val Asp Tyr Lys Ser
    450                 455                 460

Phe Lys Ala Thr Trp Thr Glu Val Val His Val Asn Arg Glu Arg Trp
465                 470                 475                 480

Arg Ala Lys Val Pro Lys Glu Glu Lys Ala Lys Lys Glu Ala Glu Glu
                485                 490                 495

Lys Ala Arg Leu Ala Ala Glu Glu Lys Gln Lys Glu Met Glu Ala Gln
            500                 505                 510

Asp Gln Thr Glu Gln Gly Lys Ala Glu Lys Lys Thr Ser Gly Glu Thr
        515                 520                 525

Lys Gly Gln Val Asn Gly Ala Arg Thr Asn Lys Gly Lys Ser Pro Lys
    530                 535                 540

Gly Asp Lys Ala Gly Glu Lys Gln Gln Asn Gly Asp Leu Lys Glu Gly
545                 550                 555                 560

Lys Asn Lys Ala Asp Lys Lys Asp His Ser Ser Thr Gly Asn Asp Ser
                565                 570                 575

Lys Lys Thr Asp Gly Thr Lys Lys Arg Ser His Asp Ser Pro Ala Pro
```

580 585 590

Ser Thr Ser Ser Thr Ser Arg Ile Thr Leu Pro Gly Asp Tyr Gly
595 600 605

<210> 24
<211> 532
<212> PRT
<213> Hamster

<400> 24

Met Asp Arg Ala Gly Cys Leu Gly Ala Gly Leu Arg Gly Leu Cys Val
1 5 10 15

Ala Ala Leu Val Leu Val Cys Ala Gly His Gly Ala Arg Arg Glu Asp
20 25 30

Gly Gly Pro Ala Cys Tyr Gly Gly Phe Asp Leu Tyr Phe Ile Leu Asp
35 40 45

Lys Ser Gly Ser Val Leu His His Trp Asn Glu Ile Tyr Tyr Phe Val
50 55 60

Glu Gln Leu Ala His Arg Phe Ile Ser Pro Gln Leu Arg Met Ser Phe
65 70 75 80

Ile Val Phe Ser Thr Arg Gly Thr Thr Leu Met Lys Leu Thr Glu Asp
85 90 95

Arg Glu Gln Ile Arg Gln Gly Leu Glu Glu Leu Gln Lys Val Leu Pro
100 105 110

Gly Gly Asp Thr Tyr Met His Glu Gly Phe Glu Arg Ala Ser Glu Gln
115 120 125

Ile Tyr Tyr Glu Asn Ser Gln Gly Tyr Arg Thr Ala Ser Val Ile Ile
130 135 140

Ala Leu Thr Asp Gly Glu Leu His Glu Asp Leu Phe Phe Tyr Ser Glu
145 150 155 160

Arg Glu Ala Asn Arg Ser Arg Asn Leu Gly Ala Ile Val Tyr Cys Val
165 170 175

Gly Val Lys Asp Phe Asn Glu Thr Gln Leu Ala Arg Ile Ala Asp Ser
180 185 190

Lys Asp His Val Phe Pro Val Asn Asp Gly Phe Gln Ala Leu Gln Gly

```
        195                 200                 205

Ile Ile His Ser Ile Leu Lys Lys Ser Cys Ile Glu Ile Leu Ala Ala
    210                 215                 220

Glu Pro Ser Thr Ile Cys Ala Gly Glu Ser Phe Gln Val Val Val Arg
225                 230                 235                 240

Gly Asn Gly Phe Arg His Ala Arg Asn Val Asp Arg Val Leu Cys Ser
                245                 250                 255

Phe Lys Ile Asn Asp Ser Val Thr Leu Asn Glu Lys Pro Phe Ala Val
            260                 265                 270

Glu Asp Thr Tyr Leu Leu Cys Pro Ala Pro Ile Leu Lys Glu Val Gly
        275                 280                 285

Met Lys Ala Ala Leu Gln Val Ser Met Asn Asp Gly Leu Ser Phe Ile
    290                 295                 300

Ser Ser Ser Val Ile Ile Thr Thr Thr His Cys Ser Asp Gly Ser Ile
305                 310                 315                 320

Leu Ala Ile Ala Leu Leu Ile Leu Phe Leu Leu Leu Ala Leu Ala Leu
                325                 330                 335

Leu Trp Trp Phe Trp Pro Leu Cys Cys Thr Val Ile Ile Lys Glu Val
            340                 345                 350

Pro Pro Pro Pro Val Glu Glu Ser Glu Glu Glu Asp Asp Asp Gly Leu
        355                 360                 365

Pro Lys Lys Lys Trp Pro Thr Val Asp Ala Ser Tyr Tyr Gly Gly Arg
    370                 375                 380

Gly Val Gly Gly Ile Lys Arg Met Glu Val Arg Trp Gly Glu Lys Gly
385                 390                 395                 400

Ser Thr Glu Glu Gly Ala Lys Leu Glu Lys Ala Lys Asn Ala Arg Val
                405                 410                 415

Lys Met Pro Glu Gln Glu Tyr Glu Phe Pro Glu Pro Arg Asn Leu Asn
            420                 425                 430

Asn Asn Met Arg Arg Pro Ser Ser Pro Arg Lys Trp Tyr Ser Pro Ile
        435                 440                 445
```

Lys Gly Lys Leu Asp Ala Leu Trp Val Leu Leu Arg Lys Gly Tyr Asp
    450                 455                 460

Arg Val Ser Val Met Arg Pro Gln Pro Gly Asp Thr Gly Arg Cys Ile
465                 470                 475                 480

Asn Phe Thr Arg Val Lys Asn Thr Gln Ala Ala Lys Tyr Pro Leu Arg
                485                 490                 495

Arg Phe Gln Arg Trp Leu His Cys Gln Asp Ala Leu Asn Gln Ile Val
            500                 505                 510

Ser His Gly Asp Gln Glu Glu Ser His Phe Arg Glu Arg Asn Ala Ala
        515                 520                 525

Leu Asp Lys Lys
    530

<210> 25
<211> 225
<212> PRT
<213> Hamster

<400> 25

Met Ala Phe Thr Ser Met Ser Thr Glu Lys Ser Lys Cys Ser Gly Arg
1               5                   10                  15

Val Arg His Lys Leu Ile Val Ile Gly Ser Pro Ser Thr Ala Leu Ala
            20                  25                  30

Pro Gly Phe Tyr Ser Gly Gln Ala Val Ser Gly Leu Gln Lys Val Val
        35                  40                  45

Phe Val Gly Leu Leu Gly Leu Leu Ile Ala Ser Gly Phe Ser Tyr Thr
    50                  55                  60

Ile Asn Ser Asn Asn Glu Ser Asn Val Gly Gly Ser Gly Pro Gln Ala
65                  70                  75                  80

Val Ser Ile Asn Asn Gln His Asn Val Ala Asn Val Asp Ser Asn Asn
                85                  90                  95

Gly Trp Gly Ser Trp Asn Ala Leu Trp Asp Tyr Glu Asn Ser Phe Ala
            100                 105                 110

Ala Thr Arg Ile Phe Ala Lys Lys Ser Cys Ile Val His Lys Met Asn
        115                 120                 125

Lys Asn Val Met Pro Ser Leu Gln Glu Leu Asp Thr Leu Val Lys Glu
    130                 135                 140

Gln Lys Asp Lys Glu Pro Glu Gly Ala Thr Pro Lys Glu Leu Met Phe
145                 150                 155                 160

Ser Ile Asn Pro Thr Arg Val Glu Asp Leu Ser Thr Phe Gly Pro Lys
                165                 170                 175

Ile Ala Gly Met Cys Gln Gly Ile Pro Thr Tyr Val Ala Glu Glu Ile
            180                 185                 190

Pro Gly Pro Asn Gln Pro Leu Tyr Ala Gln Lys Cys Phe Thr Ala Asn
        195                 200                 205

Ile Leu Trp Ile Ile Lys Leu Ser Phe Cys Gly Thr Ser Glu Glu Thr
    210                 215                 220

Tyr
225

<210> 26
<211> 307
<212> PRT
<213> Hamster

<400> 26

Met Ala Met Asp Gln Ser Thr Arg Asp Ala Arg His Pro Ser Val Thr
1               5                   10                  15

Val Arg Pro Ala Asp Ala Ala Leu Pro Ala Val Ala Ala Leu Leu Ala
            20                  25                  30

Glu Thr Val Arg Pro Ala Asp Val Glu Ser Thr Glu Asp Ala Ala Asp
        35                  40                  45

Pro Arg Val Asn Val Gln Asp Pro Glu Ala Ala Leu Leu Ser Ala Pro
    50                  55                  60

His Ser Cys Phe Arg Cys Gln Leu Leu Tyr Trp Val Gly Ala Leu Met
65                  70                  75                  80

Leu Leu Leu Gly Val Val Cys Val Pro Ile Ala Leu Phe Thr Arg Ile
                85                  90                  95

Pro Thr Arg Pro Ala Leu Thr Val Thr Thr Ser Pro Thr Pro Gly Asn
            100                 105                 110

```
Ser Glu Lys Pro Ser Asn Leu Thr Val Thr Pro Val Pro Arg Asn Ser
        115                 120                 125

Cys Pro Lys Ala Thr Gly Gln Gly Ser Ser Val Phe Ala Ile Leu Gln
    130                 135                 140

Ala Lys Glu Glu Val Lys Leu Glu Pro Asn Arg Ile Leu Lys Trp His
145                 150                 155                 160

Ser Gln Glu Gly Ala Gly Ile Ser Lys Pro Leu Gln Gly Leu Arg Tyr
                165                 170                 175

Asp Asn Val Thr Asn Glu Leu Val Val Asn Glu Ser Gly Leu Tyr Tyr
            180                 185                 190

Val Ile Leu Gln Leu Lys Leu Arg Pro Val Leu Lys Asn Thr Asp Arg
        195                 200                 205

Lys Val Arg Gly Gln Val Ser Leu Val Leu Gln Leu Asn Pro Pro Ile
    210                 215                 220

Glu Arg Pro Asp Asn Leu Ala Leu Thr Val Asp Leu Phe Pro Cys Ser
225                 230                 235                 240

Met Glu Thr Asn Leu Val Glu Gly Ser Trp Ser His Leu Ile Pro Leu
                245                 250                 255

Lys Ala Asp Asp Arg Leu Ser Val Asn Leu Arg Ala Tyr Leu Tyr Gly
            260                 265                 270

Ala Gln Glu Ala Tyr Lys Asp Trp Glu Leu Ser Gln Thr Ala Ile Thr
        275                 280                 285

Ser Phe Val Leu Phe Leu Val Pro Thr Asp Thr Pro Gln Glu Leu Pro
    290                 295                 300

Ser Ile Arg
305


<210>  27
<211>  1026
<212>  PRT
<213>  Hamster

<400>  27

Met Thr Ala Ile Lys Met Leu Gln Gly Ser Phe Pro Val Phe Leu Leu
1               5                   10                  15
```

```
Gly Ala Leu Leu Gly Val Leu His Ala Gln Gln Gln Glu Val Ile Ser
            20                  25                  30

Pro Asp Ile Ser Thr Ile Asp Arg Asn Asn Asn Cys Pro Glu Lys Ala
        35                  40                  45

Asp Cys Pro Val Asn Val Tyr Phe Val Leu Asp Thr Ser Glu Ser Val
    50                  55                  60

Ala Met Gln Ser Pro Thr Asp Ser Leu Leu Tyr His Met Gln Gln Phe
65                  70                  75                  80

Val Pro Gln Phe Ile Ser Gln Leu Gln Asn Glu Phe Tyr Leu Asp Gln
                85                  90                  95

Val Ala Leu Ser Trp Arg Tyr Gly Gly Leu His Phe Ser Asp Gln Val
            100                 105                 110

Glu Val Phe Ser Pro Pro Gly Ser Asp Arg Ala Ser Phe Thr Lys Ser
        115                 120                 125

Leu Gln Ser Ile Arg Ser Phe Arg Arg Gly Thr Phe Thr Asp Cys Ala
    130                 135                 140

Leu Ala Asn Met Thr Gln Gln Ile Arg Gln His Val Gly Arg Gly Val
145                 150                 155                 160

Val Asn Phe Ala Val Val Ile Thr Asp Gly His Val Thr Gly Asn Pro
                165                 170                 175

Cys Gly Gly Ile Lys Met Gln Ala Glu Arg Ala Arg Glu Glu Gly Ile
            180                 185                 190

Arg Leu Phe Ala Val Ala Pro Asn Arg Asn Leu Asn Glu Gln Gly Leu
        195                 200                 205

Arg Asp Ile Ala Asn Thr Pro His Glu Leu Tyr Arg Asn Asn Tyr Ala
    210                 215                 220

Thr Met Arg Pro Asp Ser Thr Glu Ile Asp Gln Asp Thr Ile Asn Arg
225                 230                 235                 240

Ile Ile Lys Val Met Lys His Glu Ala Tyr Gly Glu Cys Tyr Lys Val
                245                 250                 255

Ser Cys Leu Glu Ile Pro Gly Pro His Gly Pro Lys Gly Tyr Arg Gly
            260                 265                 270
```

```
Gln Lys Gly Ala Lys Gly Asn Met Gly Glu Pro Gly Glu Pro Gly Gln
        275                 280                 285

Lys Gly Arg Gln Gly Asp Pro Gly Ile Glu Gly Pro Ile Gly Phe Pro
    290                 295                 300

Gly Pro Lys Gly Val Pro Gly Phe Lys Gly Glu Lys Gly Glu Phe Gly
305                 310                 315                 320

Ser Asp Gly Arg Lys Gly Ala Pro Gly Leu Ala Gly Lys Asn Gly Thr
                325                 330                 335

Asp Gly Gln Lys Gly Lys Leu Gly Arg Ile Gly Pro Pro Gly Cys Lys
            340                 345                 350

Gly Asp Pro Gly Ser Arg Gly Pro Asp Gly Tyr Pro Gly Glu Ala Gly
        355                 360                 365

Thr Pro Gly Glu Gln Gly Asp Gln Gly Ala Lys Gly Asp Ser Gly Arg
    370                 375                 380

Pro Gly Arg Arg Gly Pro Pro Gly Asp Pro Gly Asp Lys Gly Ser Lys
385                 390                 395                 400

Gly Tyr Gln Gly Asn Asn Gly Ala Pro Gly Ser Pro Gly Val Lys Gly
                405                 410                 415

Gly Lys Gly Gly Pro Gly Pro Arg Gly Pro Lys Gly Glu Pro Gly Arg
            420                 425                 430

Arg Gly Asp Pro Gly Thr Lys Gly Gly Pro Gly Thr Asp Gly Pro Lys
        435                 440                 445

Gly Glu Lys Gly Asp Pro Gly Pro Glu Gly Pro Arg Gly Leu Ala Gly
    450                 455                 460

Glu Val Gly Ser Lys Gly Ala Lys Gly Asp Arg Gly Leu Pro Gly Pro
465                 470                 475                 480

Arg Gly Pro Gln Gly Ala Leu Gly Glu Leu Gly Lys Gln Gly Ser Arg
                485                 490                 495

Gly Asp Pro Gly Asp Ala Gly Pro Arg Gly Asp Ser Gly Gln Pro Gly
            500                 505                 510

Pro Lys Gly Asp Pro Gly Arg Pro Gly Phe Ser Tyr Pro Gly Pro Arg
        515                 520                 525
```

```
Gly Thr Pro Gly Glu Lys Gly Glu Pro Gly Pro Pro Gly Pro Glu Gly
    530                 535                 540

Gly Arg Gly Asp Phe Gly Leu Lys Gly Ala Pro Gly Arg Lys Gly Asp
545                 550                 555                 560

Lys Gly Glu Pro Ala Asp Pro Gly Pro Pro Gly Glu Pro Gly Pro Arg
                565                 570                 575

Gly Pro Arg Gly Ile Pro Gly Pro Glu Gly Glu Pro Gly Pro Pro Gly
            580                 585                 590

Asp Pro Gly Leu Thr Glu Cys Asp Val Met Thr Tyr Val Arg Glu Thr
        595                 600                 605

Cys Gly Cys Cys Asp Cys Glu Lys Arg Cys Gly Ala Leu Asp Val Val
    610                 615                 620

Phe Val Ile Asp Ser Ser Glu Ser Ile Gly Tyr Thr Asn Phe Thr Leu
625                 630                 635                 640

Glu Lys Asn Phe Val Ile Asn Val Val Asn Arg Leu Gly Ala Ile Ala
                645                 650                 655

Lys Asp Pro Lys Ser Glu Thr Gly Thr Arg Val Gly Val Val Gln Tyr
            660                 665                 670

Ser His Glu Gly Thr Phe Glu Ala Ile Arg Leu Asp Asp Glu Arg Val
        675                 680                 685

Asn Ser Leu Ser Ser Phe Lys Glu Ala Val Lys Asn Leu Glu Trp Ile
    690                 695                 700

Ala Gly Gly Thr Trp Thr Pro Ser Ala Leu Lys Phe Ala Tyr Asn Gln
705                 710                 715                 720

Leu Ile Lys Glu Ser Arg Arg Gln Lys Thr Arg Val Phe Ala Val Val
                725                 730                 735

Ile Thr Asp Gly Arg His Asp Pro Arg Asp Asp Asp Leu Asn Leu Arg
            740                 745                 750

Ala Leu Cys Asp Arg Asp Val Thr Val Thr Ala Ile Gly Ile Gly Asp
        755                 760                 765

Met Phe His Glu Thr His Glu Ser Glu Asn Leu Tyr Ser Ile Ala Cys
```

770 775 780

Asp Lys Pro Gln Gln Val Arg Asn Met Thr Leu Phe Ser Asp Leu Val
785 790 795 800

Ala Glu Lys Phe Ile Asp Asp Met Glu Asp Val Leu Cys Pro Asp Pro
805 810 815

Gln Ile Val Cys Pro Glu Leu Pro Cys Gln Thr Glu Leu Tyr Val Ala
820 825 830

Gln Cys Thr Gln Arg Pro Val Asp Ile Val Phe Leu Leu Asp Gly Ser
835 840 845

Glu Arg Leu Gly Glu Gln Asn Phe His Lys Ala Arg Arg Phe Val Glu
850 855 860

Glu Val Ser Arg Arg Leu Thr Leu Ala Arg Lys Asp Asp Asp Pro Leu
865 870 875 880

Asn Ala Arg Met Ala Leu Leu Gln Tyr Gly Ser Gln Asn Gln Gln Gln
885 890 895

Val Val Phe Pro Leu Thr Tyr Asn Leu Thr Thr Ile His Glu Ala Leu
900 905 910

Glu Arg Thr Ala Tyr Leu Asn Ser Phe Ser His Val Gly Ala Gly Ile
915 920 925

Val His Ala Ile Asn Asn Val Val Arg Gly Ala Arg Gly Gly Ala Arg
930 935 940

Arg His Ala Glu Leu Ser Phe Val Phe Leu Thr Asp Gly Val Thr Gly
945 950 955 960

Asn Asp Ser Leu Glu Glu Ser Val His Ser Met Arg Lys Gln Asn Val
965 970 975

Val Pro Thr Val Val Ala Val Gly Ser Asp Val Asp Met Asp Val Leu
980 985 990

Thr Lys Ile Ser Leu Gly Asp Arg Ala Ala Ile Phe Arg Glu Lys Asp
995 1000 1005

Phe Asp Ser Leu Ala Gln Pro Ser Phe Phe Asp Arg Phe Ile Arg
1010 1015 1020

```
Trp Ile  Cys
    1025


<210>  28
<211>  17
<212>  DNA
<213>  Artificial Sequence

<220>
<223>  Probe for qPCR

<400>  28
caatctccac cagcgac                                                    17


<210>  29
<211>  20
<212>  DNA
<213>  Artificial Sequence

<220>
<223>  Forward Primer for qPCR

<400>  29
gacccctgca tggtctttga                                                 20


<210>  30
<211>  22
<212>  DNA
<213>  Artificial Sequence

<220>
<223>  Reverse Primer for qPCR

<400>  30
accgtaatgc tgactcccaa gt                                              22
```